# FASCICULE DE BREVET EUROPEEN

(11) **EP 2 252 278 B1**
(45) Date de publication et mention de la délivrance du brevet: **26.10.2011**
(21) Numéro de dépôt: 09720431.7
(22) Date de dépôt: 16.01.2009
(51) Int. Cl.: A61K 31/132, A61K 31/137, A61K 31/165, A61K 31/216, A61K 31/44, A61K 31/4704, A61K 31/522, A61P 25/02, A61K 31/136, A61K 31/13, A61P 29/02

(54) **AGONISTES BÉTA-2 ADRÉNERGIQUES POUR UTILISATION DANS LE TRAITEMENT DE L'ALLODYNIE NEUROPATHIQUE CHRONIQUE**
BETA-2 ADRENERGISCHE AGONISTEN ZUR BEHANDLUNG VON CHRONISCHER NEUROPATISCHER ALLODYNIE
BETA-2 ADRENERGIC AGONISTS FOR USE IN THE TREATMENT OF CHRONIC NEUROPATHIC ALLODYNIA

(30) Priorité: 18.01.2008 FR 0800273; 26.02.2008 EP 08003458; 14.11.2008 EP 08291065
(43) Date de publication de la demande: 24.11.2010
(73) Titulaire: Centre National de la Recherche Scientifique - CNRS, 75794 Paris Cedex 16 (FR)
(72) Inventeur: BARROT, Michel, F-67000 Strasbourg (FR); YALCIN, Ipek, F-67800 Bischheim (FR); FREUND-MERCIER, Marie-José, F-67000 Strasbourg (FR); BENBOUZID, Malika, F-67000 Strasbourg (FR); CHOUCAIR-JAAFAR, Nada, F-67100 Strasbourg (FR)
(74) Mandataire: Novagraaf Technologies
(86) Numéro de dépôt international: PCT/FR2009/000045
(87) Numéro de publication internationale: WO 2009/112674

(56) Documents cités:
- EP-A- 1 813 285
- WO-A-2004/045592
- WO-A-2005/089741
- WO-A-2006/027579
- WO-A-2007/000334
- WO-A-2007/025613
- US-A1- 2005 049 256
- KODAMA DAISUKE ET AL: "Altered hippocampal long-term potentiation after peripheral nerve injury in mice" EUROPEAN JOURNAL OF PHARMACOLOGY, vol. 574, no. 2-3, novembre 2007 (2007-11), pages 127-132, XP002490043 ISSN: 0014-2999

## Description

### Domaine technique de l'invention

La présente invention se rapporte à l'utilisation comme principes actifs de composés qui sont des agonistes de récepteurs béta adrénergiques pour la fabrication de médicaments destinés au traitement des douleurs neuropathiques, notamment l'allodynie neuropathique chronique

Le principal domaine d'application de la présente invention est biomédical et plus précisément thérapeutique. Elle trouve une application en clinique humaine et vétérinaire.

La présente invention vise notamment à fournir des médicaments qui peuvent se substituer aux traitements actuellement utilisés pour traiter la douleur.

Dans la description qui suit, les références entre parenthèse (x) renvoient à la liste des références située à la fin des exemples.

### Art antérieur

D'après une étude clinique récente (Bouhassira et coll., Pain, 2008, 136:380-387 **(1)**), les douleurs chroniques à composante neuropathique ont en France une prévalence de 6,9 % dans la population générale, ce qui touche en France environ 4 millions de personnes.

Les douleurs neuropathiques sont des maladies en général chroniques liées à un dysfonctionnement ou à une lésion du système nerveux comme décrit dans Merksey et Bogduk (1994) **(2).** Elles impliquent des modifications moléculaires durables. Les traitements efficaces à long terme passent également par une plasticité du système nerveux : ils sont inefficaces les premiers jours, puis soulagent la douleur.

Le traitement des douleurs neuropathiques chroniques est difficile à prendre en charge cliniquement car la plupart des traitements antalgiques classiques sont inefficaces sur la durée. C'est notamment le cas des opiacés pour lesquels une tolérance aux effets se développe.

Le traitement pharmacologique des douleurs neuropathiques persistantes utilise actuellement principalement soit des anticonvulsivants, par exemple de la prégabaline ou de la gabapentine ; soit des antidépresseurs de type tricyclique, par exemple de l'amitriptyline, de la nortriptyline, de la clomipramine, de l'imipramine, de la désipramine, ou des antidépresseurs de type inhibiteur mixte de la recapture de la sérotonine et de la noradrénaline, par exemple de la duloxétine, de la venlafaxine. Actuellement, anticonvulsivants et antidépresseurs sont les 2 options de prescription recommandées dans divers pays comme première ligne de traitement pour lutter contre les douleurs neuropathiques (McQuay et coll., 1996, Pain 68:217-227 **(3) ;** Hempenstall et coll. PLoS Med, 2005, 2:e164 **(4) ;** Gilron et coll., CMAJ, 2006, 175:265-275 **(5) ;** Attal et coll., European Journal of Neurology, 2006, 13:1153-1169 **(6) ;** Moulin et coll., Pain Research Management, 2007, 12:13-21 **(7) ;** Dworkin et coll., Pain, 2007, 132:237-251 **(8)**).
Le document EP1813285 décrit un agoniste alpha-2 adrénergique en combinaison avec un agoniste béta-2 adrénergique pour le traitement de la douleur neuropathique.
Les documents WO 2005/08741 et WO 2006/027579 décrivent l'utilisation de béta-aminoalcools pour le traitement de maladies et de douleurs inflammatoires.

Comme pour la plupart des maladies chroniques touchant le système nerveux, aucun médicament disponible n'est à la fois efficace et supporté chez l'ensemble les patients.

Par exemple, si les antidépresseurs sont efficaces, leur utilisation pose en effet certains problèmes :
1) Des effets psychologiques : certains patients douloureux acceptent mal la prescription d'un "antidépresseur" connu pour soigner des troubles psychiatriques alors qu'ils ne sont pas dépressifs ;
2) Des effets secondaires qui peuvent être mal supportés et qui affectent environ 30% des patients (McQuay et coll., 1996 **(3)**) : par exemple une somnolence ou une sédation, des troubles de la vision, une constipation, une rétention urinaire ou une dysurie, une bouche sèche, une hypersudation, une prise de poids, une tachycardie ou une arythmie cardiaque, une hypotension orthostatique, de l'impuissance, des tremblements, des crises convulsives, de la confusion mentale, une augmentation de l'agressivité, de la paranoïa et des comportements suicidaires, une augmentation du volume mammaire, une galactorrhée, des réactions allergiques cutanées, une dysarthrie, une éosinophilie, une leucopénie, une agranulocytose, une thrombopénie, des syncopes ;
3) Un manque de spécificité d'action : L'antidépresseur en augmentant les taux extracellulaires de monoamines, (comme adrénaline, noradrénaline et sérotonine) agit sur l'ensemble des adrénorécepteurs ainsi que sur les récepteurs sérotonergiques, que ces différents récepteurs aient ou non une influence sur la douleur neuropathique ;
4) Une variabilité de l'action entre les patients : les antidépresseurs ne sont efficaces que sur une fraction des patients (entre 30 et 50% des patients) (McQuay, 1996 **(3) ;** Attal et coll., 2006 **(6)**).

Il est essentiel pour le clinicien de disposer d'une palette plus diversifiée de médicaments que celle actuellement connue de façon à proposer au patient celui qui est le mieux adapté à son cas.

Il existe donc un réel besoin de nouvelles molécules utilisables pour le traitement de la douleur, plus efficaces, mieux tolérées, mieux acceptées et ayant une action plus ciblée.

D'autres traitements de la douleur neuropathique sont utilisés actuellement, par exemple les techniques de neurostimulation, les techniques de chirurgie, les techniques d'acupuncture et la psychanalyse. Mais ces techniques nécessitent l'intervention répétée du/des praticiens et éventuellement une hospitalisation, elles sont difficiles à mettre en oeuvre, longues et/ou pas toujours satisfaisantes.

### Exposé de l'invention

La présente invention répond précisément à ce besoin et résout les inconvénients de l'art antérieur.

Depuis plus de 30 ans que les antidépresseurs sont utilisés en clinique pour soulager les douleurs neuropathiques, il n'avait encore jamais été envisagé que des agonistes des récepteurs béta-adrénergiques puissent avoir le même effet et puissent se substituer aux antidépresseurs pour soulager durablement la douleur neuropathique. Cette situation résulte sans doute de la méconnaissance du mécanisme d'action précis des antidépresseurs sur la douleur que l'on supposait relié aux alpha2-adrénorécepteurs. En effet, en prise aiguë, les agonistes des alpha2-adrénorécepteurs peuvent faciliter l'analgésie et sont utilisés en routine comme thérapie associée lors de la prise en charge de la douleur en péri-opératoire (Crassous et coll., 2007, Current Topics in Medicinal Chemistry 7:187-194 **(9)**). Il semblait donc simple et naturel d'imaginer qu'ils étaient aussi responsables de l'effet des antidépresseurs, bien que cela n'ait jamais été prouvé. Nous avons démontré que ce n'était pas le cas et qu'en réalité les adrénorécepteurs cruciaux pour l'effet thérapeutique des antidépresseurs sur la douleur neuropathique sont les béta2-adrénorécepteurs.

Les inventeurs de la présente ont identifié les adrénorécepteurs responsables de l'effet thérapeutique des antidépresseurs contre la douleur neuropathique chronique. Il s'agit des adrénorécepteurs de la famille béta, et notamment ceux du sous-type béta2. Dans un modèle murin de douleur neuropathique, ils ont en effet fait disparaître l'action thérapeutique des antidépresseurs en bloquant les adrénorécepteurs béta ou béta2. De plus, la seule administration de divers agonistes béta2-adrénergiques ou béta-adrénergiques suffit à soulager l'allodynie neuropathique dans ce modèle. L'allodynie, qui est une sensation douloureuse en réponse à un stimulus qui ne devrait pas être douloureux, est un symptôme important des douleurs neuropathiques. Les agonistes béta2-adrénergiques, et de façon plus large les agonistes béta-adrénergiques, peuvent donc permettre de traiter les douleurs neuropathiques et offrent une alternative plus ciblée à l'utilisation actuelle des antidépresseurs.

Aussi, la présente invention se rapporte à un agoniste béta-2 adrénergique comme principe actif pour la fabrication d'un médicament destiné au traitement de l'allodynie neuropathique chronique, en d'autres termes pour utilisation dans le traitement de l'allodynie neuropathique chronique, ledit agoniste béta-2 adrénergique étant administré de manière chronique.

Par « douleur neuropathique » on entend les douleurs liées à un dysfonctionnement et/ou à une lésion de l'un quelconque des éléments constitutifs des voies nociceptives comme décrit dans Merksey et Bogduk (1994) (2). Cela comprend, par exemple, les douleurs consécutives aux lésions ou irritations des nerfs périphériques (qu'il y ait une atteinte axonale et/ou myélinique), de la moelle épinière, des structures supraspinales. La lésion ou le dysfonctionnement peut être par exemple d'origine mécanique, par exemple post-traumatique ; post-chirurgical ; par exemple lié à lésion nerveuse sous une cicatrice, comme c'est le cas dans un syndrome post-thoracotomie ; à une algohallucinose dans une amputation de membre ; à une compression de nerfs dans les syndromes canalaires ; à une compression radiculaire dans une radiculopathie par hernie discale, situation qui peut être accompagnée d'une composante inflammatoire ; à une section ou un traumatisme de la moelle donnant les douleurs des paraplégies ; par exemple d'origine métabolique, par exemple neuropathie de l'alcoolisme, du diabète, des dysthyroïdies ; neuropathies carentielles ; par exemple d'origine ischémique, par exemple lié à une ischémie artérielle périphérique, une ischémie spinale, un accident vasculaire cérébral ; par exemple d'origine toxique, par exemple liée à des médicaments, par exemple les chimiothérapies anticancéreuses à base de dérivés de la pervenche, de sels de platine ; à l'amiodarone ; à des toxiques industrielles, par exemple l'acrylamide, les colles, etc. ; par exemple d'origine infectieurse, par exemple HIV, herpès, varicelle, zona ; par exemple d'origine immuno-allergique, par exemple les neuropathies paranéoplasiques, le syndrome de Guillain-Barré ; par exemple d'origine héréditaire, par exemple les neuropathies à fibres fines.

Selon l'invention, l'agoniste béta2 adrénergique peut être une molécule moins spécifique possédant entre autres une action agoniste béta2 adrénergique.

Selon l'invention, l'agoniste béta-2 adrénergique peut être choisi par exemple dans le groupe comprenant bambutérol, bitolterol, clenbutérol, fénotérol, formotérol, isoprotérénol (ou isoprénaline), levalbutérol, métaprotérénol, pirbutérol, procatérol, réprotérol, ritodrine, salbutamol (ou albutérol), salmétérol, terbutaline, ou tulobutérol. Par agoniste, on entend également dans la présente, les sels pharmaceutiquement acceptables des agonistes précités et/ou des mélanges de ces agonistes et/ou de leurs sels pharmaceutiquement acceptables.

Des sels pharmaceutiquement acceptables au sens de la présente invention sont par exemple des chlorhydrates, des sulfates, des bromures, des hydrates de bromures, des hydrates (par exemple dihydrates, trihydrates, tétrahydrates et autres), des sodiums, des fumarates, des tartrates, des mésylates, des nitrates, des dinitrates, des maléates, des acétates, des citrates, des propionates, des xinafoates ou hydroxynafoates.

Selon l'invention, le médicament peut être à usage humain ou vétérinaire.

Selon l'invention, le médicament peut être sous toute forme appropriée pour pouvoir être administré à un patient ou à un animal. L'administration peut être réalisée directement, c'est-à-dire à partir de l'agoniste pur ou sensiblement pur ou d'un sel pharmaceutiquement acceptable de l'agoniste pur ou sensiblement pur, ou en mélange avec un support pharmaceutiquement acceptable ou dans un milieu pharmaceutiquement acceptable. Le médicament peut donc être l'agoniste, ou un sel de celui-ci, sous forme pure ou sensiblement pure, ou l'agoniste dans un solvant approprié ou encore sous une forme galénique appropriée à son administration à un patient ou un animal et à son absorption par un patient ou un animal.

Le médicament peut être par exemple sous une forme choisie dans le groupe comprenant une forme injectable (par exemple, VENTOLINE (marque déposée)), un sirop (par exemple, ATARAX (marque déposée)), une solution buvable (par exemple, EFFERALGAN (marque déposée) 3%), un comprimé (par exemple, ASPIRINE DU RHONE (marque déposée)), un comprimé sécable (par exemple, CLARADOL (marque déposée)), un comprimé pelliculé (par exemple, APRANAX (marque déposée)), un comprimé pelliculé sécable (par exemple, ZYRTECSET (marque déposée)), un comprimé gastrorésistant (par exemple, ASPIRINE (marque déposée) PH 8 500 mg cp gastrorésis), un comprimé enrobé (par exemple FERVEX (marque déposée)), un comprimé dispersable (par exemple, SPASMOCALM (marque déposée)), un comprimé à croquer (par exemple, RENNIE (marque déposée)), une plaquette thermoformée (par exemple VENTODISKS (marque déposée)), une gélule (par exemple, POLYPRINE (marque déposée) gél), un effervescent (par exemple, EFFERALGAN (marque déposée)), un comprimé effervescent sécable (par exemple, PREDNISOLONE ARROW (marque déposée)), une poudre pour solution buvable (par exemple, DOLIPRANE (marque déposée)), des granules pour suspension buvable (par exemple, APRANAX (marque déposée)), une suspension pour inhalation (par exemple, AIROMIR AUTOHALER (marque déposée)), une poudre pour inhalation (par exemple, ASMELOR NOVALIZER (marque déposée)), une solution pour inhalation par nébuliseur (par exemple, SALBUTAMOL ARROW (marque déposée)), une poudre pour inhalation en gélule, (par exemple, FORADIL (marque déposée)), un suppositoire (par exemple, DOLIPRANE (marque déposée)), un collyre (par exemple, RIFAMYCINE CHIBRET (marque déposée)), une crème (par exemple, FUCIDINE (marque déposée) 2%), une pommade (par exemple, MUPIDERM (marque déposée)), un gel (par exemple, FINACEA (marque déposée)), un spray (par exemple, NASONEX (marque déposée)), un dispositif transdermique ou patch (par exemple, DUROGESIC (marque déposée)), une pastille (par exemple, STREPSILS (marque déposée)), une solution pour perfusion intraveineuse (par exemple SALBUTAMOL MERCK (marque déposée)), pour administration péridurale ou intra- ou péri-articulaire ou périphérique ou régionale ou paravertébrale (par exemple LIDOCAINE ADRENALINE AGUETANT (marque déposée) 2% SOLUTION INJECTABLE), ou administration intrathécale (par exemple BUPIFORAN (marque déposée) 0,25% SOLUTION INJECTABLE), ou infiltration, par exemple par infiltration locale (par exemple BUPIVACAINE AGUETTANT (marque déposée) 0,25% SOLUTION INJECTABLE). Dans ces exemples, l'agoniste peut être ajouté ou remplacer les principes actifs des médicaments cités. Les compositions de chacun de ces médicaments peuvent être trouvées par exemple dans le Dictionnaire VIDAL, Edition 2007.

Le support pharmaceutiquement acceptable peut être par exemple tout support pharmaceutiquement acceptable connu utilisé pour l'administration des agonistes précités à un humain ou à un animal, suivant l'usage du médicament. Par exemple, le support peut être choisi dans le groupe comprenant la povidone, la silice colloïdale, le talc, la cellulose microcristalline, le lactose monohydraté, la gélatine, la lécithine, l'amidon, la crospovidone, le glycérol, la paraffine, la butylhydroxyanisole, la vaseline, la lanoline, la gomme arabique.

Le milieu pharmaceutiquement acceptable peut être tout milieu connu qui convient à l'administration d'un agoniste tel que défini dans la présente à un humain ou à un animal. Ce milieu peut être par exemple de l'éthanol, du saccharose, du glycérol, du propylèneglycol, de la saccharine sodique, de l'acétate de sodium, de l'acide acétique, de l'eau, une solution physiologique, de la paraffine liquide, de la butylhydroxyanisole, de la vaseline, de la lanoline, de la gomme arabique.

Par « administration chronique » ou « traitement chronique », on entend dans la présente une prise répétée ou maintenue sur au moins plusieurs jours, par exemple plusieurs semaines, par exemple plusieurs mois, par exemple plusieurs années. Par exemple, une administration chronique peut consister en plusieurs prises ou administration du médicament sous l'une quelconque de ses formes décrites ci-dessus ; ou en une prise unique ou répétée du médicament sous une forme à durée d'action longue ou à durée d'action maintenue dans le temps, comme par exemple une perfusion ou un dispositif transdermique. Par exemple, une administration chronique peut conduire à une, deux, trois ou plus, prises ou administrations par jour, par exemple pendant un jour, deux jours, trois jours, etc., par exemple une semaine, deux semaines trois semaines, etc., par exemple un mois, deux mois, trois mois, etc., par exemple un an, deux ans, trois ans, etc.

Selon l'invention, le médicament peut comprendre toute dose d'agoniste pharmaceutiquement acceptable et efficace dans la mise en oeuvre de la présente invention. Par exemple, le médicament peut comprendre une dose permettant une administration, par exemple chez l'homme allant de 0,01 à 120mg/jour, par exemple de 0,01 à 20 mg/jour. Cela peut par exemple comprendre des doses de 10 à 20 mg/jour pour le bambutérol, ou de 0,02 à 0,2mg/jour pour le clenbutérol, ou de 0,05 à 0,8mg/jour pour le fénotérol, ou de 0,01 à 0,05mg/jour pour le formotérol, ou de 0,2 à 10mg/jour pour l'isoprotérénol (ou isoprénaline), ou de 0,2 à 3mg/jour pour le pirbutérol, ou de 0,05 à 72mg/kg pour la ritodrine, par exemple de 0,05 à 120mg/jour, ou de 0,1 à 36mg/jour pour le salbutamol, ou de 0,025 à 0,1 mg/jour pour le salmétérol, de 0,5 à 13,5mg/jour pour la terbutaline, 0,5 à 60 mg/jour pour le métaprotérénol, de 0,3 à 4mg/jour pour le lévalbutérol, de 0,5 à 12mg/jour pour le réprotérol, de 1 à 6mg/jour pour le tulobutérol, de 0,5 à 10mg/jour pour le bitoltérol. Il s'agit bien entendu d'exemples, les doses pourront être adaptées notamment en fonction de la sensibilité des patients à ce traitement et du mode d'administration.

La présente invention **décrit** également un procédé de traitement de l'allodynie neuropathique chronique, ce procédé comprenant l'administration à un patient d'un agoniste tel que défini ci-dessus. Le patient peut être humain ou animal. Des agonistes utilisables et des formulations sont donnés en exemple ci-dessus.

L'administration peut être réalisée par tout moyen connu de l'homme du métier, en particulier par tout moyen connu pour l'administration des agonistes précités. Des exemples de modes d'administration sont décrits ci-dessus.

Par exemple, l'administration peut être réalisée par injection directe de l'agoniste, ou par perfusion, par prise orale sous forme notamment de sirop, de solution buvable, de comprimé, de comprimé effervescent, ou, pelliculé, ou sécable, ou enrobé, ou gastrorésistant, ou dispersable, ou à croquer, de tablette thermoformée, de gélule, de pastille, de poudre ou de granules pour solution buvable, par utilisation d'une suspension ou d'une poudre pour inhalation, d'une poudre pour inhalation en gélule, d'une solution pour inhalation par nébuliseur, par une prise de suppositoire, par utilisation de collyre, par utilisation de crème, de pomade ou de gel, par utilisation de spray, par utilisation de dispositifs transdermiques, par administration ou infiltration ou perfusion péridurale ou intra- ou péri-articulaire ou régionale ou périphérique ou intrathécale ou paravertébrale ou locale. Des exemples de compositions utilisables pour ces différentes voies d'administration sont décrits ci-dessus.

L'administration peut être définie de manière à permettre une délivrance pharmaceutiquement acceptable et efficace pour traiter la douleur, en particulier la douleur neuropathique. Par exemple, l'administration peut comprendre une dose par exemple chez l'homme allant de 0,01 à 120mg/jour, par exemple de 0,01 à 20 mg/jour. Cela peut par exemple comprendre des doses de 10 à 20 mg/jour pour le bambutérol, ou de 0,02 à 0,2mg/jour pour le clenbutérol, ou de 0,05 à 0,8mg/jour pour le fénotérol, ou de 0,01 à 0,05mg/jour pour le formotérol, ou de 0,2 à 10mg/jour pour l'isoprotérénol (ou isoprénaline), ou de 0,2 à 3mg/jour pour le pirbutérol, ou de 0,05 à 72mg/kg pour la ritodrine, par exemple de 0,05 à 120mg/jour, ou de 0,1 à 36mg/jour pour le salbutamol, ou de 0,025 à 0,1 mg/jour pour le salmétérol, de 0,5 à 13,5mg/jour pour la terbutaline. Il s'agit bien entendu d'exemples, les doses pourront être adaptées, notamment en fonction de la sensibilité des patients à ce traitement.

L'administration peut être réalisée en une seule fois ou en plusieurs fois.

L'administration d'agonistes béta-adrénergiques selon le procédé décrit dans la présente est une administration chronique.

Les doses d'agonistes béta-adrénergiques administrées, de façon chronique, peuvent être comprises entre 0,01 et 20mg/jour, par exemples entre 0,05 et 15mg/jour, par exemple entre 0,05 et 10mg/jour, par exemple entre 0,1 et 5mg/jour, ou encore par exemple entre 0,15 et 350 microg/kg et par jour, par exemple entre 0,8 à 250 microg/kg et par jour, par exemple entre 0,8 à 170 microg/kg et par jour, par exemple entre 1,5 à 90 microg/kg et par jour.

L'action des agonistes béta2-adrénergiques dans l'utilisation conforme à la présente invention est plus précise au niveau moléculaire que celle des antidépresseurs pour traiter l'allodynie neuropathique chronique car ces agonistes peuvent agir directement sur le récepteur responsable de l'effet thérapeutique sans affecter les autres récepteurs adrénergiques. Cette action plus ciblée réduit certainement les effets secondaires indésirables.

La présente invention **décrit également** l'utilisation. d'agonistes béta-adrénergiques pour le traitement de l'allodynie neuropathique chronique de longue durée. En effet, les données expérimentales obtenues par les inventeurs à partir de leur modèle animal de douleur neuropathique, notamment d'allodynie neuropathique, extrêmement proche de la clinique humaine, montrent clairement une amélioration importante des symptômes douloureux après traitement avec de telles molécules.

De plus, ces molécules sont déjà utilisées en clinique humaine pour traiter d'autres affections ce qui facilite et surtout rend plus rapides les premiers essais thérapeutiques sur les douleurs neuropathiques. Les perspectives thérapeutiques sont donc immédiates. De nombreuses molécules agonistes béta ou béta2-adrenergiques ont déjà obtenu une autorisation de mise sur le marché et sont couramment utilisées dans le cadre d'autres pathologies, essentiellement les crises d'asthme ou les maladies pulmonaires obstructives chroniques, ou pour inhiber les contractions utérines. Mais ces molécules n'ont encore jamais été testées contre les l'allodynie neuropathique chronique. L'existence de ces autorisations fait que les tests cliniques sur les effets secondaires ont déjà été réalisés et devrait potentiellement favoriser/accélérer à moindre coût le passage aux essais et à l'utilisation clinique dans le cadre des douleurs neuropathiques.

Par ailleurs, l'efficacité d'un traitement chronique est fortement dépendante de l'acceptation et du suivi du traitement chronique par le patient. Psychologiquement, il peut être plus facile d'accepter d'être traité par une molécule connue pour être prescrite contre l'asthme que par une molécule à visée psychiatrique connue pour être prescrite contre la dépression.

D'autres avantages pourront apparaître à l'homme du métier à la lecture des exemples qui suivent, donnés bien entendu à titre illustratif et non-limitatif en référence aux figures annexées.

### Brève description des figures

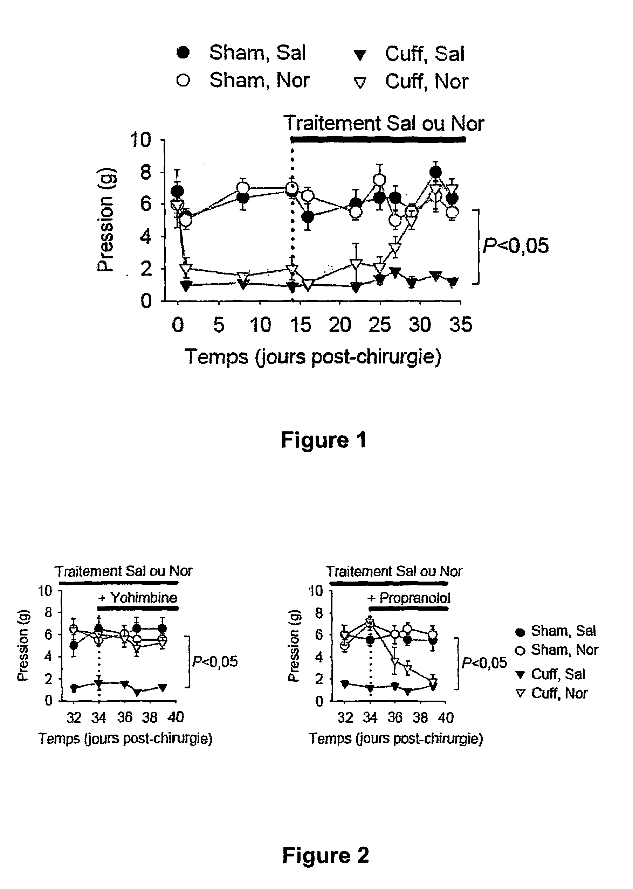

La figure 1 est un graphe montrant l'effet d'une solution saline (« Sal ») ou de Nortriptyline (« Nor ») sur des souris témoins (« Sham ») sans manchon ou neuropathiques avec manchon (« Cuff ») décrites dans les exemples 1 et 2. Sur l'axe des ordonnées, la pression exercée est exprimée en grammes (g) et sur l'axe des abscisses, le temps est exprimé en jours à compter de l'opération chirurgicale exposée dans l'exemple 1.

La figure 2 est un graphe montrant chez des animaux témoins (« Sham ») ou neuropathiques (« Cuff ») l'effet d'un co-traitement par une solution saline (« Sal ») ou de Nortriptyline (« Nor ») et par l'antagoniste alpha2-adrénergique yohimbine ou l'antagoniste béta-adrénergique propranolol, décrit dans l'exemple 3. Sur l'axe des ordonnées, la pression exercée est exprimée en grammes (g) et sur l'axe des abscisses, le temps est exprimé en jours à compter de l'opération chirurgicale exposée dans l'exemple 1.

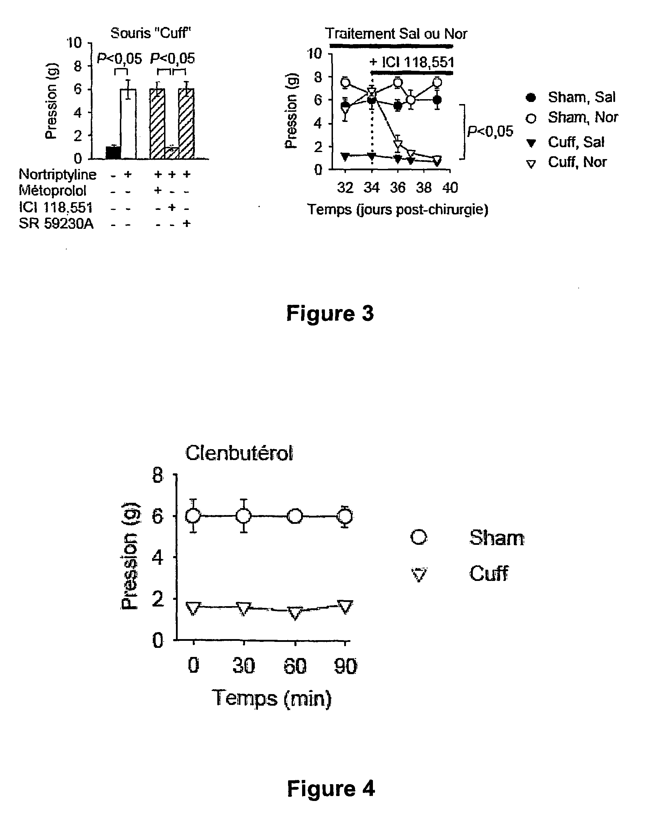

La figure 3 comprend 2 graphes. Le premier montre chez des animaux neuropathiques l'effet d'un co-traitement de Nortriptyline et de l'antagoniste béta1-adrénergique métoprolol, de l'antagoniste béta2-adrénergique ICI 118,551 ou de l'antagoniste béta3-adrénergique SR59230A, décrit dans les exemples 4, 5 et 6. Sur l'axe des ordonnées, la pression exercée est exprimée en grammes (g). Le second graphe détaille l'effet d'un co-traitement de Nortriptyline et de l'antagoniste béta2-adrénergique ICI 118,551, décrit dans l'exemple 5. Sur l'axe des ordonnées, la pression exercée est exprimée en grammes (g) et sur l'axe des abscisses, le temps est exprimé en jours à compter de l'opération chirurgicale exposée dans l'exemple 1.

La figure 4 est un graphe montrant chez des animaux témoins (« Sham ») ou neuropathiques (« Cuff ») l'effet de la première injection (« injection aiguë ») de clenbutérol décrit dans l'exemple 7. Sur l'axe des ordonnées, la pression exercée est exprimée en grammes (g) et sur l'axe des abscisses, le temps est exprimé en minutes après l'injection.

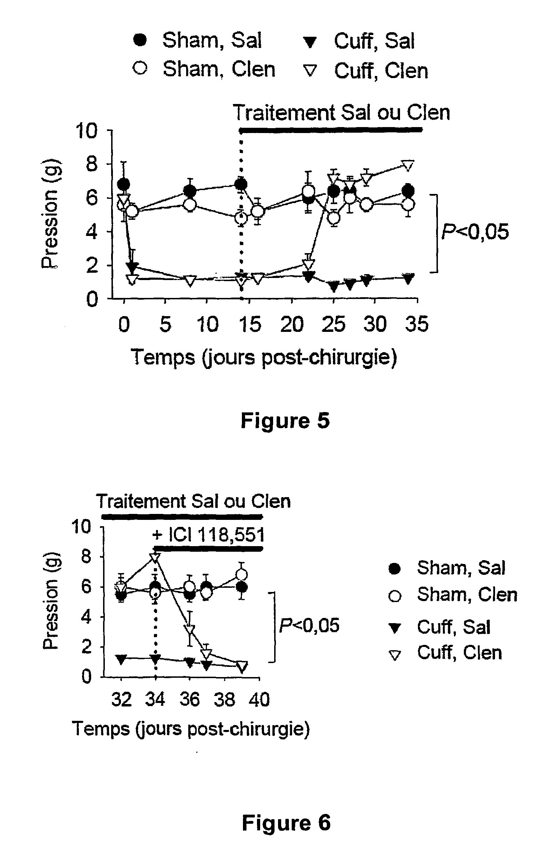

La figure 5 est un graphe montrant chez des animaux témoins (« Sham ») ou neuropathiques (« Cuff ») l'effet d'un traitement au clenbutérol (« Clen ») décrit dans l'exemple 7. Sur l'axe des ordonnées, la pression exercée est exprimée en grammes (g) et sur l'axe des abscisses, le temps est exprimé en jours à compter de l'opération chirurgicale exposée dans l'exemple 1.

La figure 6 est un graphe montrant chez des animaux témoins (« Sham ») ou neuropathiques (« Cuff ») l'effet d'un co-traitement de clenbutérol et de l'antagoniste béta2-adrénergique ICI 118,551 décrit dans l'exemple 8. Sur l'axe des ordonnées, la pression exercée est exprimée en grammes (g) et sur l'axe des abscisses, le temps est exprimé en jours à compter de l'opération chirurgicale exposée dans l'exemple 1.

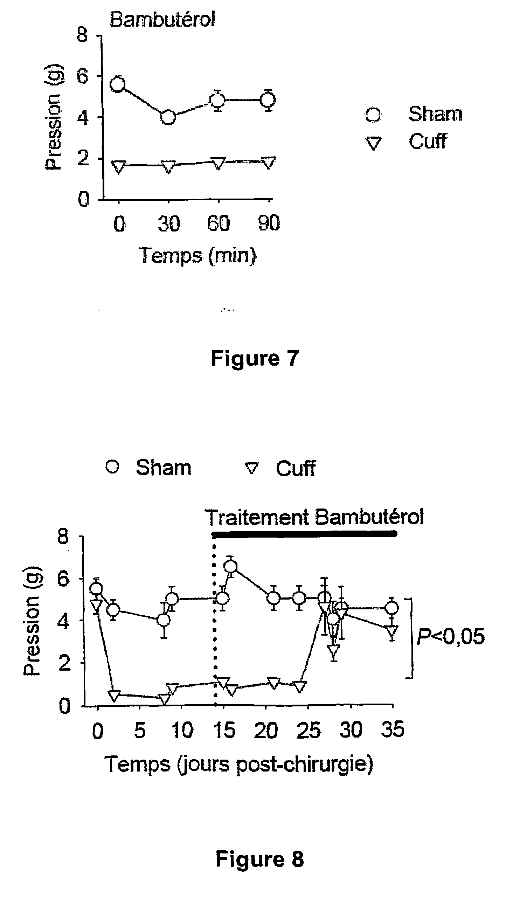

La figure 7 est un graphe montrant chez des animaux témoins (« Sham ») ou neuropathiques (« Cuff ») l'effet de la première injection (« injection aiguë ») de bambutérol décrit dans l'exemple 9. Sur l'axe des ordonnées, la pression exercée est exprimée en grammes (g) et sur l'axe des abscisses, le temps est exprimé en minutes après l'injection.

La figure 8 est un graphe montrant chez des animaux témoins (« Sham ») ou neuropathiques (« Cuff ») l'effet d'un traitement au bambutérol décrit dans l'exemple 9. Sur l'axe des ordonnées, la pression exercée est exprimée en grammes (g) et sur l'axe des abscisses, le temps est exprimé en jours à compter de l'opération chirurgicale exposée dans l'exemple 1.

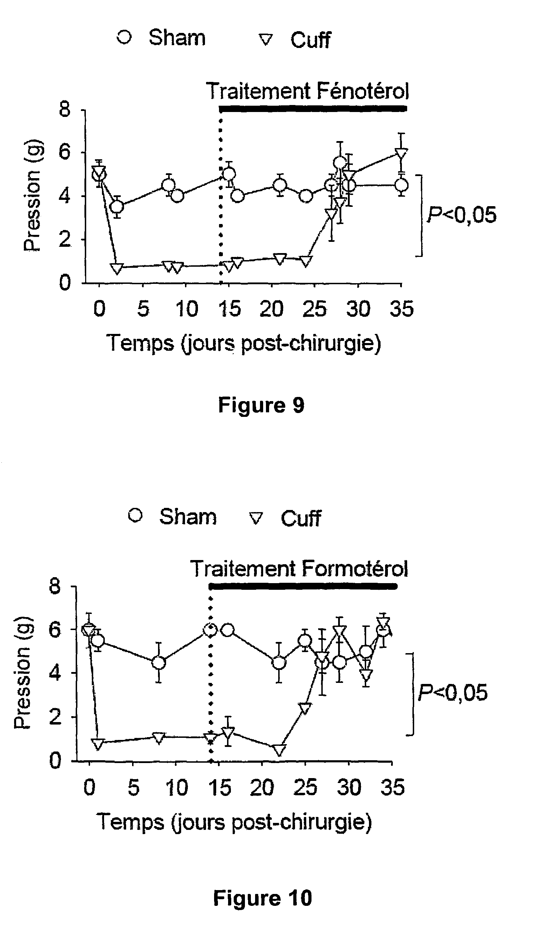

La figure 9 est un graphe montrant chez des animaux témoins (« Sham ») ou neuropathiques (« Cuff ») l'effet d'un traitement au fénotérol, décrit dans l'exemple 10. Sur l'axe des ordonnées, la pression exercée est exprimée en grammes (g) et sur l'axe des abscisses, le temps est exprimé en jours à compter de l'opération chirurgicale exposée dans l'exemple 1.

La figure 10 est un graphe montrant chez des animaux témoins (« Sham ») ou neuropathiques (« Cuff ») l'effet d'un traitement au formotérol à la dose 0,5 mg/kg décrit dans l'exemple 11. Sur l'axe des ordonnées, la pression exercée est exprimée en grammes (g) et sur l'axe des abscisses, le temps est exprimé en jours à compter de l'opération chirurgicale exposée dans l'exemple 1.

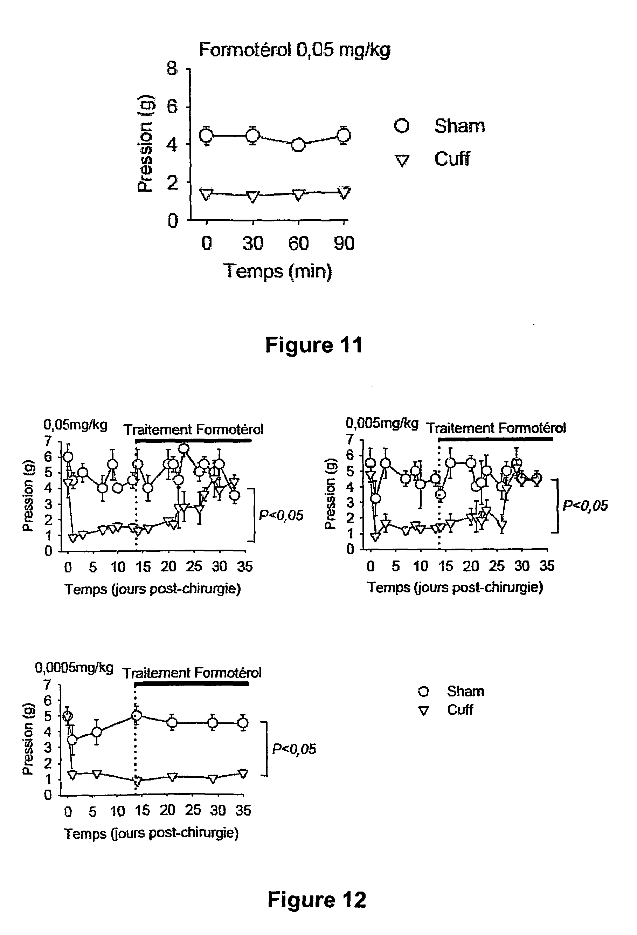

La figure 11 est un graphe montrant chez des animaux témoins (« Sham ») ou neuropathiques (« Cuff ») l'effet de la première injection (« injection aiguë ») de formotérol à la dose 0,05 mg/kg décrit dans l'exemple 11. Sur l'axe des ordonnées, la pression exercée est exprimée en grammes (g) et sur l'axe des abscisses, le temps est exprimé en minutes après l'injection.

La figure 12 est un graphe montrant chez des animaux témoins (« Sham ») ou neuropathiques (« Cuff ») l'effet de traitements au formotérol à différentes doses (0,05 mg/kg ; 0,005 mg/kg ; 0,0005 mg/kg) décrits dans l'exemple 11. Sur l'axe des ordonnées, la pression exercée est exprimée en grammes (g) et sur l'axe des abscisses, le temps est exprimé en jours à compter de l'opération chirurgicale exposée dans l'exemple 1.

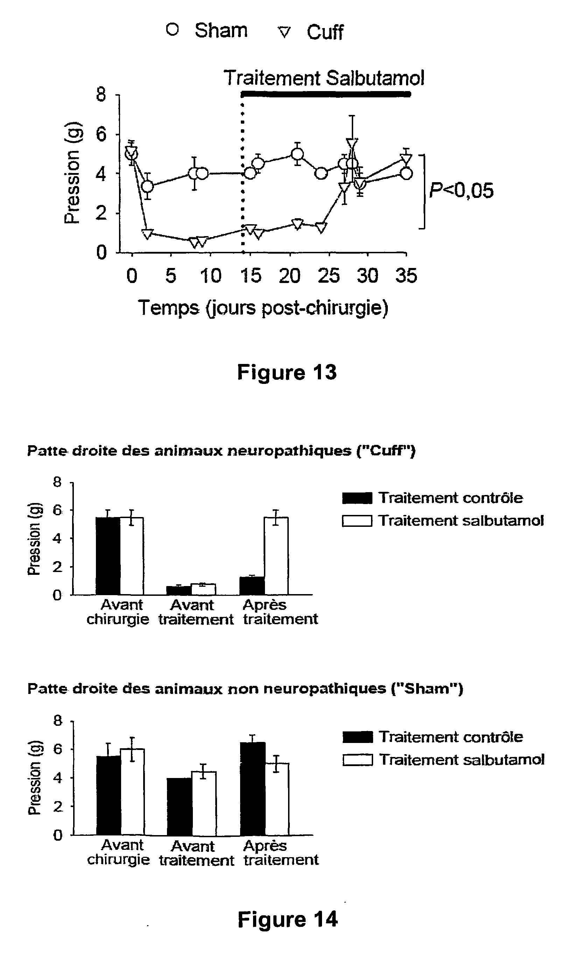

La figure 13 est un graphe montrant chez des animaux témoins (« Sham ») ou neuropathiques (« Cuff ») l'effet d'un traitement au salbutamol décrit dans l'exemple 12. Sur l'axe des ordonnées, la pression exercée est exprimée en grammes (g) et sur l'axe des abscisses, le temps est exprimé en jours à compter de l'opération chirurgicale exposée dans l'exemple 1.

La figure 14 est un graphe montrant chez des animaux témoins (« Sham ») ou neuropathiques (« Cuff ») l'effet d'un traitement au salbutamol par inhalation décrit dans l'exemple 12. Sur l'axe des ordonnées, la pression exercée est exprimée en grammes (g). Sur l'axe des abscisses, le temps est présenté est exprimé en jours avant chirurgie comme exposée dans l'exemple 1, avant le début du traitement et à la fin du traitement comme décrits dans l'exemple 12.

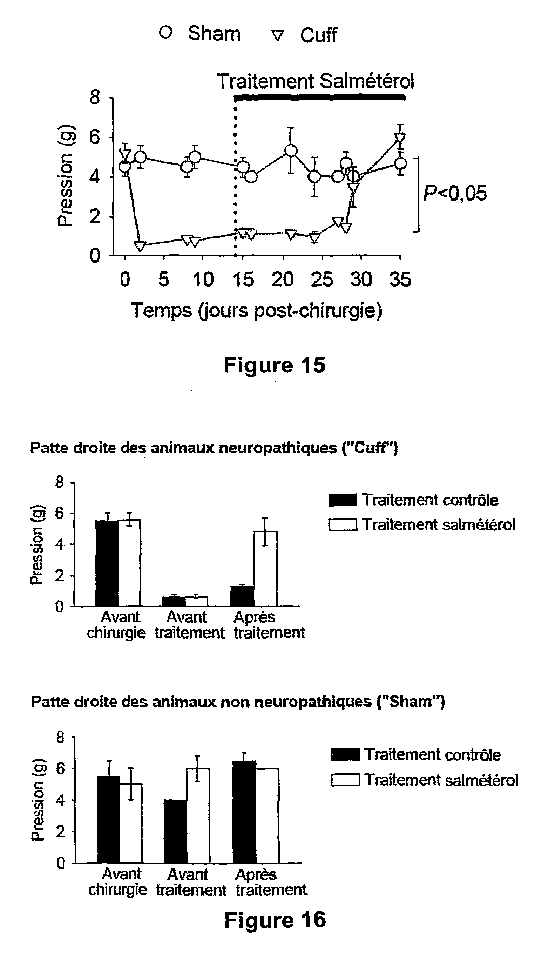

La figure 15 est un graphe montrant chez des animaux témoins (« Sham ») ou neuropathiques (« Cuff ») l'effet d'un traitement au salmétérol décrit dans l'exemple 13. Sur l'axe des ordonnées, la pression exercée est exprimée en grammes (g) et sur l'axe des abscisses, le temps est exprimé en jours à compter de l'opération chirurgicale exposée dans l'exemple 1.

La figure 16 est un graphe montrant chez des animaux témoins (« Sham ») ou neuropathiques (« Cuff ») l'effet d'un traitement au salmétérol par inhalation décrit dans l'exemple 13. Sur l'axe des ordonnées, la pression exercée est exprimée en grammes (g). Sur l'axe des abscisses, le temps est présenté est exprimé en jours avant chirurgie comme exposée dans l'exemple 1, avant le début du traitement et à la fin du traitement comme décrits dans l'exemple 12.

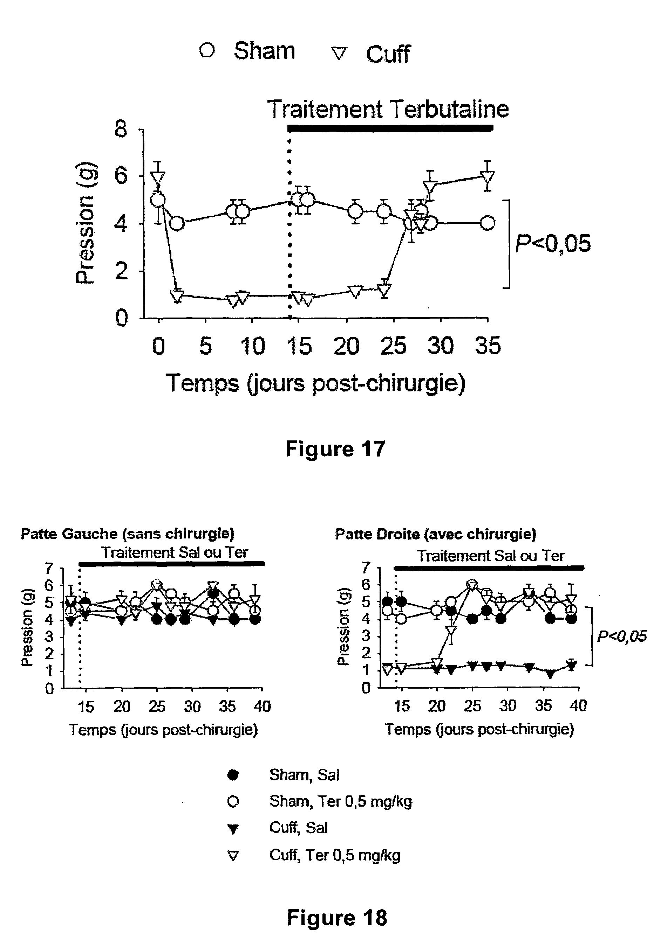

La figure 17 est un graphe montrant chez des animaux témoins (« Sham ») ou neuropathiques (« Cuff ») l'effet d'un traitement à la terbutaline décrit dans l'exemple 14. Sur l'axe des ordonnées, la pression exercée est exprimée en grammes (g) et sur l'axe des abscisses, le temps est exprimé en jours à compter de l'opération chirurgicale exposée dans l'exemple 1.

La figure 18 est un graphe montrant chez des animaux témoins (« Sham ») ou neuropathiques (« Cuff ») l'effet d'un traitement à la terbutaline (0,5 mg/kg) ou à la saline décrit dans l'exemple 14. Le graphe de gauche présente les résultats les résultats de la patte gauche (sans chirurgie). Celui de droite présente les résultats de la patte droite (avec chirurgie). Sur l'axe des ordonnées, la pression exercée est exprimée en grammes (g) et sur l'axe des abscisses, le temps est exprimé en jours à compter de l'opération chirurgicale exposée dans l'exemple 1.

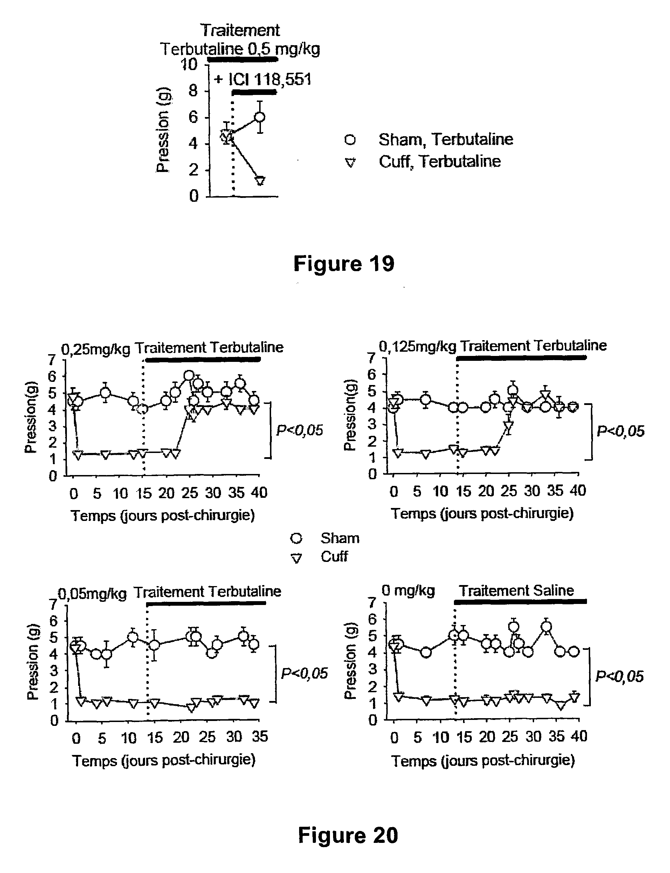

La figure 19 est un graphe montrant chez des animaux témoins (« Sham ») ou neuropathiques (« Cuff ») l'effet d'un co-traitement de terbutaline à la dose 0,5 mg/kg et de l'antagoniste béta2-adrénergique ICI 118,551 décrit dans l'exemple 14. Sur l'axe des ordonnées, la pression exercée est exprimée en grammes (g).

La figure 20 est un graphe montrant chez des animaux témoins, (« Sham ») ou neuropathiques (« Cuff ») l'effet de traitements à la terbutaline à différentes doses (0,25 mg/kg ; 0,125 mg/kg ; 0,05 mg/kg ; 0 mg/kg) décrits dans l'exemple 14. Sur l'axe des ordonnées, la pression exercée est exprimée en grammes (g) et sur l'axe des abscisses, le temps est exprimé en jours à compter de l'opération chirurgicale exposée dans l'exemple 1.

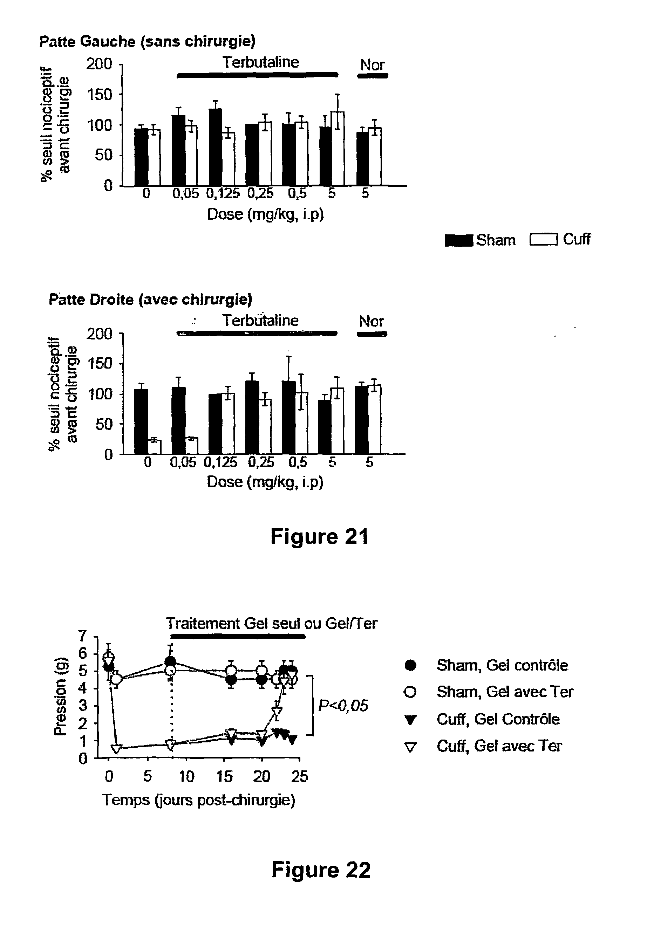

La figure 21 est un graphe récapitulant chez des animaux témoins (« Sham ») ou neuropathiques (« Cuff ») l'effet comparé d'un traitement chronique à la solution contrôle (saline, « dose 0 »), à la terbutaline à différentes doses et à la nortriptyline décrit dans l'exemple 14. Le graphe du haut présente les résultats de la patte gauche (sans chirurgie). Celui du bas présente les résultats de la patte droite (avec chirurgie). Sur l'axe des ordonnées, la récupération des animaux est indiquée en pourcentage de leur sensibilité avant chirurgie.

La figure 22 est un graphe montrant chez des animaux témoins (« Sham ») ou neuropathiques (« Cuff ») l'effet d'un traitement à la terbutaline (Ter) par gel décrit dans l'exemple 14. Le gel contrôle ne contient pas de terbutaline. Sur l'axe des ordonnées, la pression exercée est exprimée en grammes (g) et sur l'axe des abscisses, le temps est exprimé en jours à compter de l'opération chirurgicale exposée dans l'exemple 1.

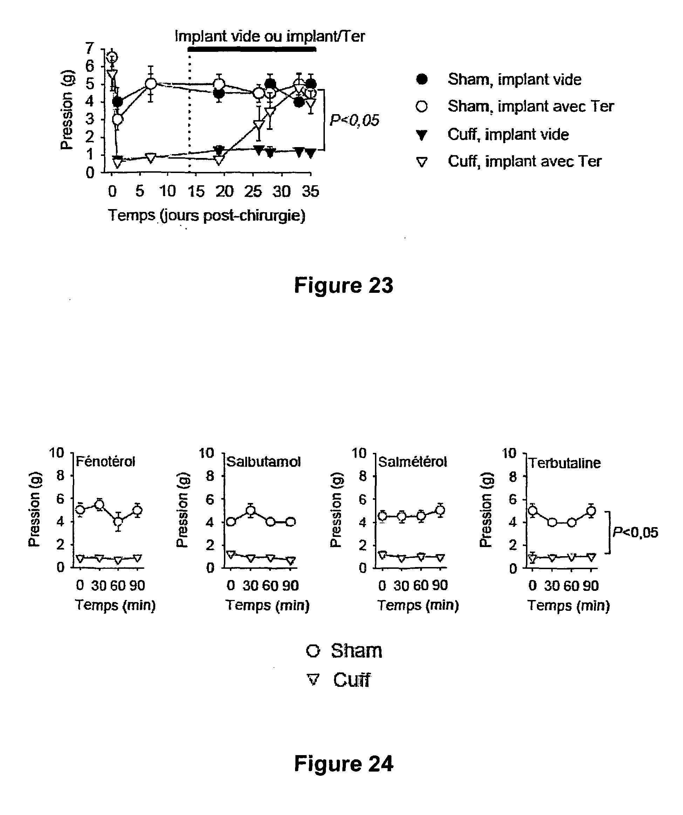

La figure 23 est un graphe montrant chez des animaux témoins (« Sham ») ou neuropathiques (« Cuff ») l'effet de la pose d'un implant de terbutaline (Ter) décrit dans l'exemple 14. L'implant contrôle ne contient pas de terbutaline. Sur l'axe des ordonnées, la pression exercée est exprimée en grammes (g) et sur l'axe des abscisses, le temps est exprimé en jours à compter de l'opération chirurgicale exposée dans l'exemple 1.

La figure 24 est un graphe montrant chez des animaux témoins (« Sham ») ou neuropathiques (« Cuff ») l'effet de la première injection (« injection aiguë ») de fénotérol, de salbutamol, de salmétérol, et de terbutaline décrit dans l'exemple 15. Sur l'axe des ordonnées, la pression exercée est exprimée en grammes (g) et sur l'axe des abscisses, le temps est exprimé en minutes après l'injection.

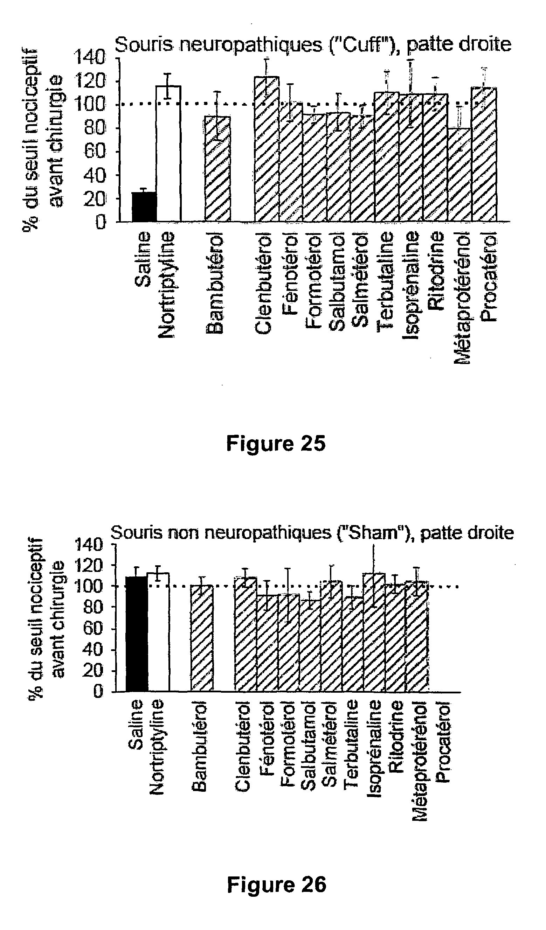

La figure 25 est un graphe récapitulant chez des animaux neuropathiques (« Cuff ») l'effet comparé d'un traitement chronique à la solution contrôle (saline), à la nortriptyline, au bambutérol, au clenbutérol, au fénotérol, au formotérol, au salbutamol, au salmétérol, à la terbutaline, à l'isoprénaline, à la ritodrine, au métaprotérénol et au procatérol décrit dans l'exemple 16. Sur l'axe des ordonnées, la récupération des animaux est indiquée en pourcentage de leur sensibilité avant chirurgie.

La figure 26 est un graphe récapitulant chez des animaux témoins (« Sham ») l'effet comparé d'un traitement chronique à la solution contrôle (saline), à la nortriptyline, au bambutérol, au clenbutérol, au fénotérol, au formotérol, au salbutamol, au salmétérol, à la terbutaline, à la l'isoprénaline, à la ritodrine, au métaprotérénol et au procatérol décrit dans l'exemple 17. Sur l'axe des ordonnées est indiqué le seuil nociceptif des animaux en pourcentage de leur sensibilité avant chirurgie.

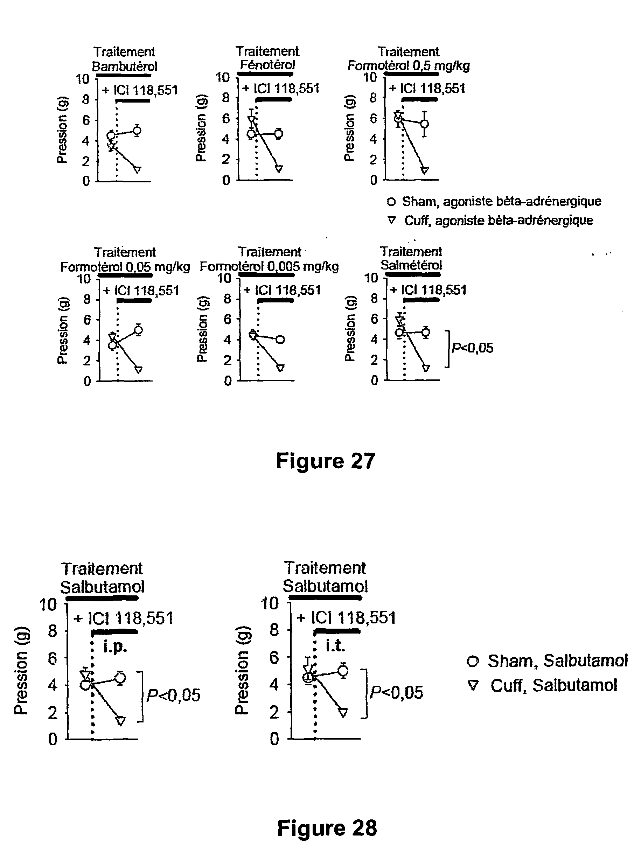

La figure 27 est un graphe montrant chez des animaux témoins (« Sham ») ou neuropathiques (« Cuff ») l'effet d'un co-traitement de bambutérol, ou de fénotérol, ou de formotérol, ou de salmétérol, et de l'antagoniste béta2-adrénergique ICI 118,551 décrit dans l'exemple 18. Sur l'axe des ordonnées, la pression exercée est exprimée en grammes (g).

La figure 28 est un graphe montrant chez des animaux témoins (« Sham ») ou neuropathiques (« Cuff ») l'effet d'un co-traitement de salbutamol et de l'antagoniste béta2-adrénergique ICI 118,551 injecté soit intrapéritonéalement soit en intrathécal, décrit dans l'exemple 19. Sur l'axe des ordonnées, la pression exercée est exprimée en grammes (g).

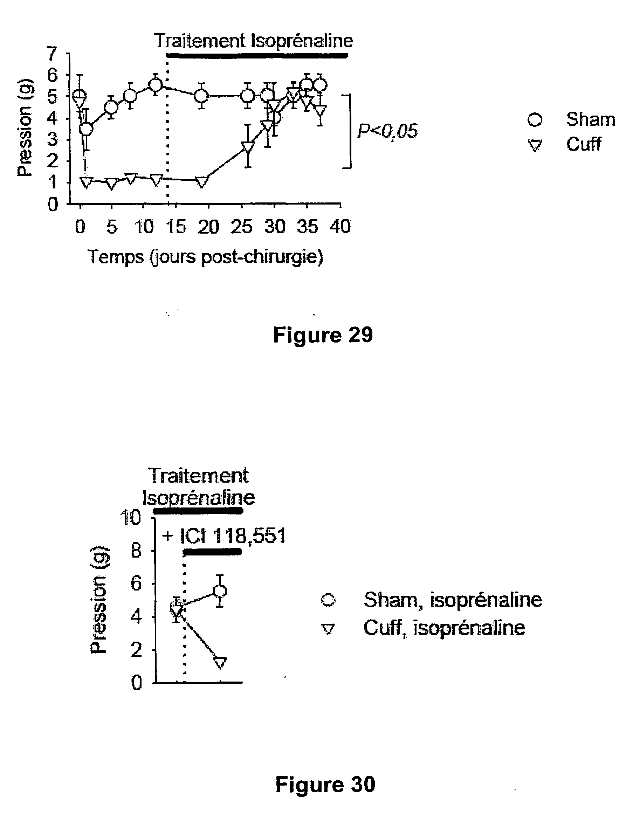

La figure 29 est un graphe montrant chez des animaux témoins (« Sham ») ou neuropathiques (« Cuff ») l'effet d'un traitement à l'isoprénaline (ou isoprotérénol) décrit dans l'exemple 20. Sur l'axe des ordonnées, la pression exercée est exprimée en grammes (g) et sur l'axe des abscisses, le temps est exprimé en jours à compter de l'opération chirurgicale exposée dans l'exemple 1.

Là figure 30 est un graphe montrant chez des animaux témoins (« Sham ») ou neuropathiques (« Cuff ») l'effet d'un co-traitement d'isoprénaline et de l'antagoniste béta2-adrénergique ICI 118,551 décrit dans l'exemple 20. Sur l'axe des ordonnées, la pression exercée est exprimée en grammes (g).

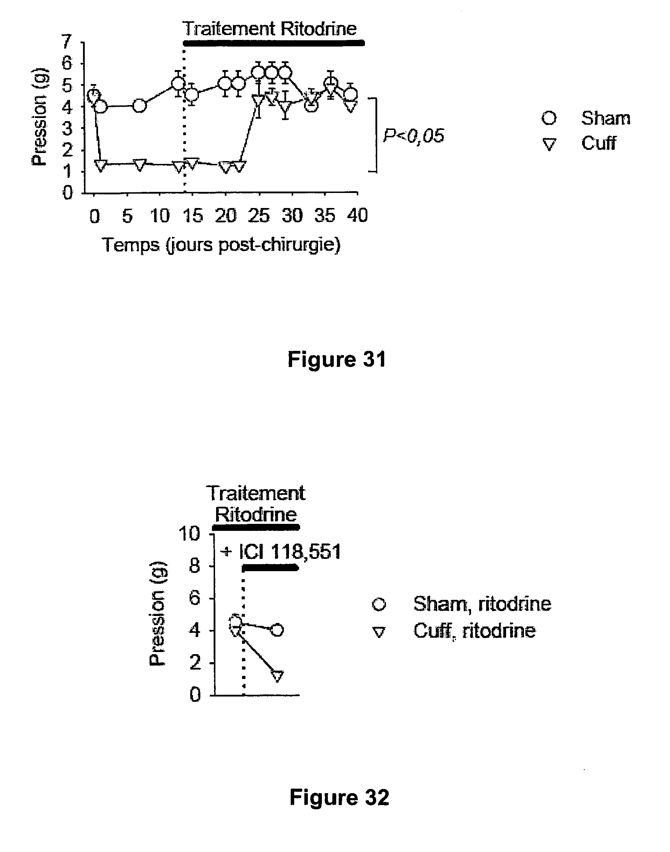

La figure 31 est un graphe montrant chez des animaux témoins (« Sham ») ou neuropathiques (« Cuff ») l'effet d'un traitement à la ritodrine décrit dans l'exemple 20. Sur l'axe des ordonnées, la pression exercée est exprimée en grammes (g) et sur l'axe des abscisses, le temps est exprimé en jours à compter de l'opération chirurgicale exposée dans l'exemple 1.

La figure 32 est un graphe montrant chez des animaux témoins (« Sham ») ou neuropathiques (« Cuff ») l'effet d'un co-traitement de ritodrine et de l'antagoniste béta2-adrénergique ICI 118,551 décrit dans l'exemple 20. Sur l'axe des ordonnées, la pression exercée est exprimée en grammes (g).

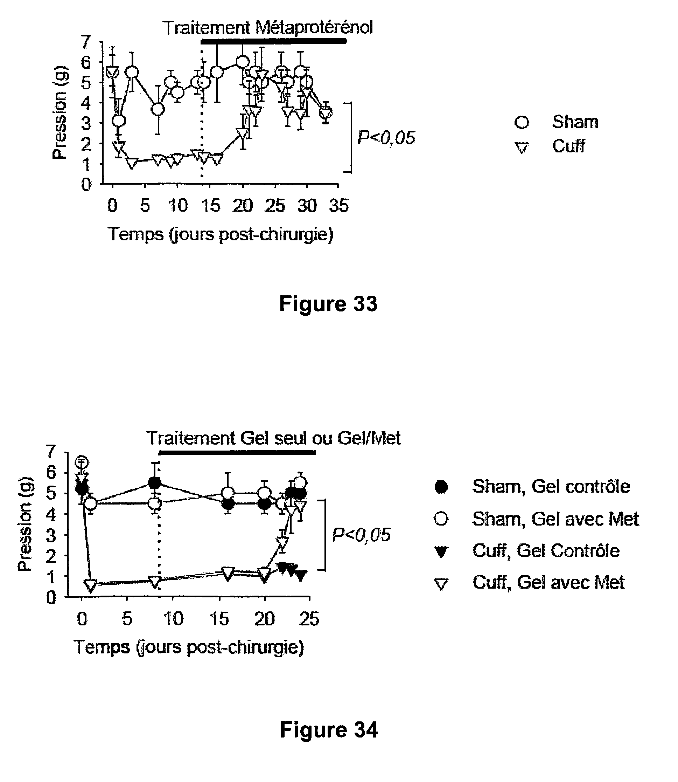

La figure 33 est un graphe montrant chez des animaux témoins (« Sham ») ou neuropathiques (« Cuff ») l'effet d'un traitement au métaprotérénol décrit dans l'exemple 20. Sur l'axe des ordonnées, la pression exercée est exprimée en grammes (g) et sur l'axe des abscisses, le temps est exprimé en jours à compter de l'opération chirurgicale exposée dans l'exemple 1.

La figure 34 est un graphe montrant chez des animaux témoins (« Sham ») ou neuropathiques (« Cuff ») l'effet d'un traitement au métaprotérénol par gel décrit dans l'exemple 20. Sur l'axe des ordonnées, la pression exercée est exprimée en grammes (g) et sur l'axe des abscisses, le temps est exprimé en jours à compter de l'opération chirurgicale exposée dans l'exemple 1.

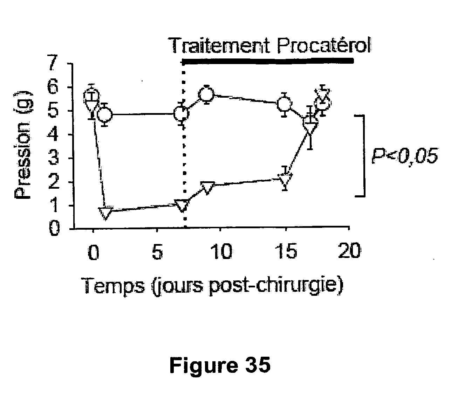

La figure 35 est un graphe montrant chez des animaux témoins (« Sham ») ou neuropathiques (« Cuff ») l'effet d'un traitement au procatérol décrit dans l'exemple 20. Sur l'axe des ordonnées, la pression exercée est exprimée en grammes (g) et sur l'axe des abscisses, le temps est exprimé en jours à compter de l'opération chirurgicale exposée dans l'exemple 1.

### EXEMPLES

Dans les exemples qui suivent, les protocoles et résultats expérimentaux illustrent les données scientifiques des inventeurs qui conduisent à proposer les agonistes béta-adrénergiques pour traiter la douleur neuropathique, notamment l'allodynie neuropathique. Les inventeurs démontrent expérimentalement notamment :
**1.** Qu'un blocage répété des récepteurs alpha2-adrénergiques (par exemple par de la yohimbine) est inefficace à bloquer l'effet antalgique des antidépresseurs : cet effet ne passe donc pas par les alpha2 adrénorécepteurs ;
**2.** Qu'un blocage répété des récepteurs béta-adrénergiques (par exemple par le propranolol) bloque l'effet thérapeutique des antidépresseurs : les béta-adrénorécepteurs sont donc responsables de l'effet thérapeutique des antidépresseurs sur la douleur neuropathique, par exemple sur l'allodynie neuropathique. Cet effet est spécifique puisque le propranolol n'affecte pas la réponse des animaux témoins, traités ou non par exemple par la nortriptyline.

Les inventeurs testent également des molécules spécifiques des 3 sous-types de béta-adrénorécepteurs : le métoprolol contre les béta1; l'ICI118,551 contre les béta2 et le SR59230A contre les béta3, et montrent que seul l'antagoniste des béta2-adrénorécepteurs bloque l'effet des antidépresseurs. Cela démontre que l'effet thérapeutique des antidépresseurs contre la douleur neuropathique, par exemple contre l'allodynie neuropathique, nécessite les béta2-adrénorécepteurs. Cet effet est spécifique puisque l'ICI118,551 n'affecte pas la réponse des animaux témoins, traités ou non par la nortriptyline.

Les inventeurs démontrent ensuite qu'un traitement chronique par divers agonistes spécifiques des béta2-adrénorécepteurs fait totalement disparaître la douleur neuropathique, par exemple l'allodynie neuropathique, et peut donc se substituer à l'utilisation des antidépresseurs. Cette démonstration a été faite par exemple avec le clenbutérol, le fenotérol, le formotérol, le salbutamol, le salmétérol, la terbutaline, la ritodrine, le métaprotérénol et le procatérol. La même démonstration a été faite avec par exemple le bambutérol et l'isoprénaline, des agonistes généraux béta-adrénergique. Les agonistes béta2-adrénergiques, et plus généralement les agonistes béta-adrénergiques, peuvent donc permettre de traiter les douleurs neuropathiques, notamment l'allodynie neuropathique.

Les inventeurs démontrent que cet effet des agonistes des béta2-adrénorécepteurs sur la douleur neuropathique, par exemple sur l'allodynie neuropathique, ne s'observe pas de façon aiguë après la première injection de l'agoniste considéré. Cela a été démontré par exemple avec le clenbutérol, le bambutérol, le fénotérol, le formotérol, le salbutamol, le salmétérol et la terbutaline. Un traitement chronique est donc nécessaire.

Les inventeurs montrent que le traitement chronique par divers agonistes spécifiques des béta2-adrénorécepteurs n'affecte pas de façon aspécifique la sensibilité des animaux témoins. Cette démonstration a été faite par exemple avec le clenbutérol, le fenotérol, le formotérol, le salbutamol, le salmétérol, la terbutaline, la ritodrine, le métaprotérénol et le procatérol. La même démonstration a été faite par exemple avec le bambutérol et l'isoprénaline, des agonistes généraux béta-adrénergique. Aux doses thérapeutiques, les agonistes béta2-adrénergiques, et plus généralement les agonistes béta-adrénergiques, n'ont donc pas à long terme d'effet aspécifique sur la sensibilité nociceptive.

Les inventeurs démontrent que l'antagoniste des béta2-adrénorécepteurs ICI118,551 bloque l'effet thérapeutique d'un traitement chronique par les agonistes des récepteurs béta-adrénergiques sur la douleur neuropathique, par exemple sur l'allodynie neuropathique. Cette démonstration a été faite par exemple avec le bambutérol, le l'isoprénaline, clenbutérol, le fénotérol, le formotérol, le salbutamol, le salmétérol, la terbutaline, la ritodrine et le procatérol. L'effet du traitement par ces agonistes sur la douleur neuropathique par exemple sur l'allodynie neuropathique, passe donc bien par leur action sur les béta2-adrénorécepteurs.

Les inventeurs démontrent que l'antagoniste des béta2-adrénorécepteurs ICI118,551 délivré par voie intrathécale bloque l'effet thérapeutique d'un traitement chronique par un agoniste des récepteurs béta-adrénergiques. Cette démonstration a été faite par exemple avec le salbutamol. Ces résultats montrent que la moelle épinière et/ou les ganglions rachidiens sont impliqués dans l'effet thérapeutique des agonistes béta2-adrénergiques.

Les inventeurs démontrent que l'administration par inhalation d'agonistes des béta2-adrénorécepteurs, comme par exemple le salbutamol ou le salmétérol, soulage l'allodynie neuropathique.

Les inventeurs démontrent que l'administration transcutanée sous forme de gel d'agonistes des béta2-adrénorécepteurs, comme par exemple la terbutaline ou le métaprotérénol, soulage l'allodynie neuropathique.

Les inventeurs démontrent que la délivrance continue d'agonistes des béta2-adrénorécepteurs, comme avec par exemple avec des implants de terbutaline, soulage l'allodynie neuropathique.

Le détail des protocoles opératoires et résultats obtenus est présenté dans les exemples ci-dessous.

### Exemple 1 : modèle murin de douleur neuropathique.

Les expérimentations ont été conduites sur des souris mâles adultes. Les expérimentations pharmacologiques ont été conduites en utilisant des souris C57BL/6J (âgées de 3 à 6 semaines à leur arrivée, Charles River, L'Arbresle, France).

Toutes les expérimentations ont débuté avec des souris âgées de 6 à 9 semaines. Pour les expériences d'application de gel, les souris ont été stabulées individuellement. Pour toutes les autres expériences les souris ont été réunies par quatre à cinq par cage et maintenues sous une alternance jour/nuit de 12 heures (lumière à 6h00 du matin) avec de la nourriture et de l'eau ad libitum. Les procédures ont été conduites suivant la Directive 86/6609/CEE.

Le modèle est obtenu par pose d'un manchon de polyéthylène autour de la branche principale du nerf sciatique droit de chaque souris. Avant toute intervention chirurgicale, les souris ont été assignées aux différents groupes expérimentaux de façon à ce que le seuil de la nociception mécanique de base et le poids soient équivalents entre les différents groupes. L'intervention chirurgicale a été réalisée sous anesthésie kétamine-xylazine (kétamine : 17 mg/mL, xylazine 2,5 mg/mL, injection intrapéritonéale à raison de 4mUkg) (Centravet Société Anonyme, Taden, France). La branche commune du nerf sciatique a été exposée et une section de 2 mm de long d'un tube creux de polyéthylène PE-20 (« Harvard Apparatus », Les Ulis, France) a été placée autour de celle-ci (groupe « Cuff »). Ce protocole a été précédemment publié (Benbouzid, M. et al. Biological Psychiatry 63:633-636 (2008) (10) et Benbouzid, M. et al. European Journal of Pain 12 :591-599 (2008) (11)). Des souris témoins ont été opérées suivant la même procédure, mais sans mettre en place le manchon (groupe « Sham »). Les groupes expérimentaux sont constitués d'au moins 4 souris pour chaque groupe témoin (« Sham ») et d'au moins 5 souris pour chaque groupe neuropathique (« Cuff »).

La pose du manchon de polyéthylène (groupe « Manchon » ou "cuff") autour de la branche principale du nerf sciatique entraîne une allodynie mécanique, c'est-à-dire une réponse à une stimulation normalement non douloureuse, par rapport aux animaux contrôles (groupe « Témoins » ou "sham"). Cela signifie que le seuil de pression entraînant une réponse de retrait de la patte de la souris diminue fortement, comme l'indique le graphe de la figure 1.

Pour les tests, dans les exemples qui suivent, la pression est exercée sur la patte des souris au moyen de filaments de von Frey (Bioseb, Chaville, France) (Benbouzid, M. et al. Biological Psychiatry 63 :633-636 (2008) **(10)** et Benbouzid, M. et al. European Journal of Pain 12 :591-599 (2008) **(11)**). Les souris sont placées dans des boites de Plexiglas (marque déposée) transparent (7 cm x 9 cm x 7 cm) posées sur une grille surélevée. Avant le test, tes souris-sont habituées 15 minutes à ce dispositif. Lors du test, les filaments de von Frey sont appliqués sur la surface plantaire de chaque patte arrière selon des forces ascendantes (de 0,16 g jusqu'à une réponse de retrait de la patte, ou jusqu'à 10 g au maximum). Chaque filament est testé 5 fois par patte et le seuil est défini par la présence de 3 retraits ou plus observés sur les 5 essais. Avant la chirurgie telle que décrite ci-dessus, les animaux ont été pré-testés pour établir leur réponse nociceptive de base. Lors des expériences de traitement chronique et celles d'injection répétées d'antagonistes, les tests sont effectués les matins avant la première injection de la journée. Lors des expériences d'injection aiguë d'agoniste, les tests sont effectués après la première injection en suivant le décours temporel tel que présenté sur les graphes.

La chirurgie pour la mise en place du manchon correspond au jour 0. L'allodynie mécanique obtenue sur ces souris dure plus de 2 mois ainsi que précédemment publié (Benbouzid, M. et al. Biological Psychiatry 63 :633-636 (2008) **(10)** et Benbouzid, M. et al. European Journal of Pain 12 :591-599 (2008) **(11)**).

Ce modèle de souris neuropathique est sensible au traitement chronique avec un antidépresseur tricyclique (nortriptyline ou amitriptyline) tel que précédemment publié (Benbouzid, M. et al. Biological Psychiatry 63 :633-636 (2008) **(10)**).

Pour tous les traitements faits par injection, celles-ci ont été faites par voie intrapéritonéale dans un volume de 50 microL pour 10 mg de poids corporel.

### Exemple 2 : Effet thérapeutique d'un antidépresseur tricyclique.

Sur des modèles de souris obtenus de la manière décrite dans l'exemple 1, lorsque l'allodynie était bien en place, au jour 15 après l'opération de mise en place du manchon, a été commencé un traitement thérapeutique par l'antidépresseur nortriptyline ou « Nor », à raison de 5 mg/kg (Nortriptyline hydrochloride ; disponible auprès de fournisseurs comme par exemple Sigma-Aldrich numéro de catalogue N7261 ; diluée dans une solution saline à 0,9% de NaCl), injection 2 fois par jour (matin et soir). Ce traitement reproduit ce qui est observé en clinique : le traitement est inefficace les premiers jours mais fait ensuite totalement disparaître l'allodynie.

Un groupe « placébo » de souris a été soumis à une injection de solution saline uniquement (solution 0,9% de NaCl), sans antidépresseur, (sur le graphique « Sal » pour : injection de solution saline à 0,9 %). Ce groupe « Sal » reste allodynique pendant toute l'expérience.

Les résultats de cet exemple sont montrés sur la figure 1 annexée.

La sensibilité nociceptive des souris du groupe témoin (ou « sham ») n'est pas affectée par le traitement par la nortriptyline : leur seuil nociceptif reste stable au long de l'expérience.

### Exemple 3 : Absence d'effet d'un blocage des alpha2-adrénorécepteurs sur l'action de l'antidépresseur et présence d'effet d'un blocage des béta-adrénorécepteurs sur l'action de l'antidépresseur.

Le graphe de gauche de la figure 2 montre que chez des animaux neuropathiques (« Manchon ») qui ont été traités pendant 3 semaines par la nortriptyline (5 mg/kg, 2 fois par jour) tel que décrit dans l'exemple 2 (et donc soulagés de leur allodynie), un co-traitement par la nortriptyline et l'antagoniste (bloqueur) des alpha2-adrénorécepteurs (la yohimbine, 2 mg/kg, intrapéritonéal) (yohimbine hydrochloride ; disponible auprès de fournisseurs comme par exemple Sigma-Aldrich numéro de catalogue Y3125 ; diluée dans une solution saline à 0,9% de NaCl) n'a aucune influence sur le bénéfice thérapeutique. L'effet thérapeutique de la nortriptyline ne passe donc pas par un recrutement des alpha2-adrénorécepteurs.

Un blocage des béta-adrénorécepteurs supprime l'effet thérapeutique de l'antidépresseur.

Le graphe de droite de la figure 2 montre que chez des animaux neuropathiques traités pendant 3 semaines par la nortriptyline (5 mg/kg, 2 fois par jour), un co-traitement par la nortriptyline tel que décrit dans l'exemple 2 et l'antagoniste des béta-adrénorécepteurs (le propranolol, 5 mg/kg, intrapéritonéal) ((+/-)-propranolol hydrochtoride ; disponible auprès de fournisseurs comme par exemple Sigma-Aldrich numéro de catalogue P0884 ; dilué dans une solution saline à 0,9% de NaCl) fait réapparaître l'allodynie en quelques jours. L'effet thérapeutique de l'antidépresseur passe donc par les béta-adrénorécepteurs.

La sensibilité nociceptive des souris du groupe témoin (ou « sham ») n'est pas affectée par les injections de yohimbine ou de propranolol : leur seuil nociceptif reste stable au long de l'expérience.

Les mêmes résultats sont obtenus en utilisant du sotatol, 2 mg/kg, ((+/-) sotalol hydrochloride ; disponible auprès de fournisseurs comme par exemple Sigma-Aldrich numéro de catalogue S0278 ; dilué dans une solution saline à 0,9% de NaCl) à la place du propranol, 5 mg/kg.

### Exemple 4 : Absence d'effet d'un blocage des béta 1-adrénorécepteurs sur l'action de l'antidépresseur.

Le graphe de gauche de la figure 3 montre que chez des animaux neuropathiques traités pendant 3 semaines par la nortriptyline tel que décrit dans l'exemple 2, un co-traitement de 6 jours par la nortriptyline et l'antagoniste des béta1-adrénorécepteurs (le métoprolol, 2 mg/kg, intrapéritonéal) ((+/-)-metoprolol (+)-tartrate ; disponible auprès de fournisseurs comme par exemple Sigma-Aldrich numéro de catalogue M5391 ; dilué dans une solution saline à 0,9% de NaCl) n'a aucune influence sur le bénéfice thérapeutique.

Les mêmes résultats sont obtenus en utilisant de l'atenolol, 5 mg/kg, (atenolol ; disponible auprès de fournisseurs comme par exemple Sigma-Aldrich numéro de catalogue A7655 ; dilué dans une solution saline à 0,9% de NaCl) à la place du métoprolol 2 mg/kg.

### Exemple 5 : Présence d'effet d'un blocage des béta2-adrénorécepteurs sur l'action de l'antidépresseur.

Le graphe de gauche de la figure 3 montre que chez des animaux neuropathiques traités pendant 3 semaines par la nortriptyline tel que décrit dans l'exemple 2, un co traitement de 6 jours par la nortriptyline et l'antagoniste des béta2-adrénorécepteurs (l'ICI 118,551, 2 mg/kg, intrapéritonéal) (ICI 118,551 hydrochloride ; disponible auprès de fournisseurs comme par exemple Sigma-Aldrich numéro de catalogue I127 ; dilué dans une solution saline à 0,9% de NaCl) entraîne une réapparition de l'allodynie neuropathique.

Un blocage des béta2-adrénorécepteurs supprime l'effet thérapeutique de l'antidépresseur.

Le graphe de droite de la figure 3 détaille le décours temporel de la réapparition de la douleur neuropathique chez les souris portant le manchon (« Cuff ») lorsque l'ICI 118,551 est co-administré avec la nortriptyline (« Nor »), tel que décrit ci-dessus. Il montre que l'allodynie réapparaît dans les 48 heures suivant le début du co-traitement.

La sensibilité nociceptive des souris du groupe témoin (ou « sham ») n'est pas affectée par les injections d'ICI 118,551.

### Exemple 6 : Absence d'effet d'un blocage des béta3-adrénorécepteurs sur l'action de l'antidépresseur.

Le graphe de gauche la figure 3 montre que chez des animaux neuropathiques traités pendant 3 semaines par la nortriptyline, un co-traitement de 6 jours par la nortriptyline et l'antagoniste des béta3-adrénorécepteurs (le SR59230A, 2,5 mg/kg, intrapéritonéal) (SR 59230A ; disponible auprès de fournisseurs comme par exemple Sigma-Aldrich numéro de catalogue S8688 ; dilué dans une solution saline à 0,9% de NaCl) n'a aucune influence sur le bénéfice thérapeutique.

### Exemple 7: THERAPIE: L'agoniste des béta2-adrénorécepteurs clenbuterol soulage l'allodynie neuropathique.

Le graphe de la figure 4 montre que la première injection de l'agoniste béta2 adrénergique clenbutérol (0,3 mg/kg, intrapéritonéal) (clenbutérol hydrochloride ; disponible auprès de fournisseurs comme par exemple Sigma-Aldrich numéro de catalogue C5423 ; dilué dans une solution saline à 0,9% de NaCl) n'a pas d'effet sur l'allodynie mécanique des souris neuropathiques (« Cuff ») depuis 2 semaines.

Les souris du groupe témoin (ou « sham ») ne sont pas non plus affectées par la première administration de cet agoniste.

Le graphe de la figure 5 montre qu'un traitement par l'agoniste béta2 adrénergique clenbuterol (« Clen », 0,3 mg/kg, intrapéritonéal, 2 fois par jour (matin et soir)) (clenbutérol hydrochloride ; disponible auprès de fournisseurs comme par exemple Sigma-Aldrich numéro de catalogue C5423 ; dilué dans une solution saline à 0,9% de NaCl) supprime totalement l'allodynie mécanique chez les souris neuropathiques (« Cuff ») après traitement chronique.

La sensibilité nociceptive des souris du groupe témoin (ou « sham ») n'est pas affectée par les injections de clenbutérol.

Ces données montrent pour la première fois que l'administration de clenbutérol peut soulager la douleur neuropathique, notamment l'allodynie neuropathique.

### Exemple 8 : Effet d'un blocage des béta2-adrénorécepteurs sur l'action du clenbutérol.

Le graphe de la figure 6 montre qu'après 3 semaines de traitement par le clenbutérol tel que décrit dans l'exemple 7, si on co-administre le clenbutérol et l'antagoniste des béta2-adrénorécepteurs ICI 118,551 (« ICl », 2 mg/kg, intrapéritonéal, tel que décrit dans l'exemple 5), cela fait réapparaître l'allodynie chez les animaux neuropathiques (« Cuff ») en quelques jours. L'effet thérapeutique du clenbutérol passe donc bien par son action sur les béta2-adrénorécepteurs.

La sensibilité nociceptive des souris du groupe témoin (ou « sham ») n'est pas affectée par les injections d'ICI 118,551.

### Exemple 9: THERAPIE: L'agoniste des béta-adrénorécepteurs bambutérol soulage l'allodynie neuropathique.

Le graphe de la figure 7 montre que la première injection de l'agoniste béta2 adrénergique bambutérol (0;5 mg/kg intrapéritoroéal) (bambutérol hydrochloride ; disponible auprès de fournisseurs comme par exemple Sigma-Aldrich numéro de catalogue B8684 ; dilué dans une solution saline à 0,9% de NaCl) n'a pas d'effet antiallodynique chez des souris neuropathiques (« Cuff ») depuis 2 semaines.

Les souris du groupe témoin (ou « sham ») ne sont pas non plus affectées par la première administration de cet agoniste.

Le graphe de la figure 8 montre qu'un traitement par l'agoniste béta adrénergique bambutérol (0,5 mg/kg, 2 fois par jour (matin et soir)) (bambutérol) hydrochloride ; disponible auprès de fournisseurs comme par exemple Sigma-Aldrich numéro de catalogue B8684 ; dilué dans une solution saline à 0,9% de NaCl) supprime totalement l'allodynie mécanique chez les souris neuropathiques (« Cuff ») après traitement chronique.

La sensibilité nociceptive des souris du groupe témoin (ou « sham ») n'est pas affectée par les injections de bambutérol.

Ces données montrent pour la première fois que l'administration de bambutérol peut soulager la douleur neuropathique, notamment l'allodynie neuropathique.

### Exemple 10 : THERAPIE : L'agoniste des béta2-adrénorécepteurs fénotérol soulage l'allodynie neuropathique.

Le graphe de la figure 9 montre qu'un traitement par l'agoniste béta2 adrénergique fénotérol (0,7 mg/kg, 2 fois par jour (matin et soir)) (fénotérol hydrobromide ; disponible auprès de fournisseurs comme par exemple Sigma-Aldrich numéro de catalogue F1016 ; dilué dans une solution saline à 0,9% de NaCl) supprime totalement l'allodynie mécanique chez les souris neuropathiques (« Cuff ») après traitement chronique.

La sensibilité nociceptive des souris du groupe témoin (ou « sham ») n'est pas affectée par les injections de fénotérol.

Ces données montrent pour la première fois que l'administration de fénotérol peut soulager la douleur neuropathique, notamment l'allodynie neuropathique.

### Exemple 11 : THERAPIE : L'agoniste des béta2-adrénorécepteurs formotérol soulage l'allodynie neuropathique.

Le graphe de la figure 10 montre qu'un traitement par l'agoniste béta2 adrénergique formotérol (0,5 mg/kg, injection 2 fois par jour (matin et soir)) (formotérol fumarate dihydrate ; disponible auprès de fournisseurs comme par exemple Sigma-Aldrich numéro de catalogue F9552 ; dilué dans une solution saline à 0,9% de NaCl) supprime totalement l'allodynie mécanique chez les souris neuropathiques (« Cuff ») après traitement chronique.

Le graphe de la figure 11 montre qu'une injection aiguë de l'agoniste béta2 adrénergique formotérol (0,05 mg/kg, intrapéritonéal) n'a pas d'effet antiallodynique chez des souris neuropathiques (« Cuff ») depuis 2 semaines.

Les souris du groupe témoin (ou « sham ») ne sont pas non plus affectées par la première administration de cet agoniste à cette dose.

Les graphes de la figure 12 montrent que des traitements par l'agoniste béta2 adrénergique formotérol aux doses 0,05 mg/kg ou 0,005 mg/kg (injection 2 fois par jour (matin et soir)) suppriment totalement l'allodynie mécanique de la patte droite (patte neuropathique) après traitement chronique. Les groupes expérimentaux sont constitués de 4 souris pour tous les groupes témoins (« Sham »), de 5 souris pour les groupes neuropathiques (« Cuff ») recevant les doses 0,05 mg/kg ou 0,005 mg/kg et de 4 souris pour le groupe neuropathique (« Cuff ») recevant la dose 0,0005 mg/kg.

La sensibilité nociceptive des souris du groupe témoin (ou « sham ») n'est pas affectée par les injections de formotérol.

Ces données montrent pour la première fois que l'administration de formotérol peut soulager la douleur neuropathique, notamment l'allodynie neuropathique.

### Exemple 12 : THERAPIE : L'agoniste des béta2-adrénorécepteurs salbutamol soulage l'allodynie neuropathique.

Le graphe de la figure 13 montre qu'un traitement par l'agoniste béta2 adrénergique salbutamol (2 mg/kg, injection 2 fois par jour (matin et soir)) (salbutamol hémisulfate ; disponible auprès de fournisseurs comme par exemple Sigma-Aldrich numéro de catalogue S5013 ; dilué dans une solution saline à 0,9% de NaCl) soulage l'allodynie mécanique chez les souris neuropathiques (« Cuff ») après traitement chronique.

La sensibilité nociceptive des souris du groupe témoin (ou « sham ») n'est pas affectée par les injections de salbutamol.

Ces données montrent pour la première fois que l'administration de salbutamol peut soulager la douleur neuropathique, notamment l'allodynie neuropathique.

Les graphes de la figure 14 montrent qu'un traitement par l'agoniste béta2 adrénergique salbutamol par inhalation (2 fois par jour (matin et soir)) (sulfate de salbutamol en inhalateur ; disponible auprès de fournisseurs comme par exemple GlaxoSmithKline sous le nom de Ventoline®) soulage l'allodynie mécanique chez les souris neuropathiques (« Cuff ») après traitement chronique. Pour effectuer ce traitement, les animaux ont été placés de façon individuelle à l'intérieur de chambres d'inhalation (disponible auprès de fournisseurs comme par exemple GlaxoSmithKline, chambre d'inhalation Babyhater® référence 7072184) dont les sorties d'air principales ont été obturées. Le flacon pressurisé de Ventoline® est connecté à la chambre et une pression permet de délivrer 100 µg de salbutamol par séance d'administration (soir 200 µg par jour) à l'intérieur de la chambre d'inhalation lorsque le museau de la souris est proche de l'inhalateur. L'animal est alors laissé 5 minutes dans la chambre avant d'être retiré et remis dans sa cage. Le traitement a débuté 2 semaines après la chirurgie de pose des manchons autour du nerf sciatique. Après 2 semaines de traitement avec une pression d'inhalateur par séance d'administration, le traitement a été passé pour 9 jours à 3 pressions d'inhalateur par séance d'administration. Le graphe de la figure 14 correspond aux résultats avant chirurgie, avant le début du traitement et au dernier jour de traitement. Les animaux contrôles pour !e traitement (animaux non-traités) suivent la même procédure mais sans délivrance de salbutamol.

Ces données montrent pour la première fois qu'une administration de salbutamol par inhalation peut soulager la douleur neuropathique, notamment l'allodynie neuropathique.

### Exemple 13 : THERAPIE: L'agoniste des béta2-adrénorécepteurs salmétérol soulage l'allodynie neuropathique.

Le graphe de la figure 15 montre qu'un traitement par l'agoniste béta2 adrénergique salmétérol (1 mg/kg, injection 2 fois par jour (matin et soir)) (salmétérol xinafoate ; disponible auprès de fournisseurs comme par exemple Sigma-Aldrich numéro de catalogue S5068 ; dilué dans une solution saline à 0,9% de NaCl) supprime totalement l'allodynie mécanique après traitement chronique.

La sensibilité nociceptive des souris du groupe témoin (ou « sham ») n'est pas affectée par les injections de salmétérol.

Ces données montrent pour la première fois que l'administration de salmétérol peut soulager la douleur neuropathique, notamment l'allodynie neuropathique.

Les graphes de la figure 16 montrent qu'un traitement par l'agoniste béta2 adrénergique salmétérol par inhalation (2 fois par jour (matin et soir)) (xinafoate de salmétérol en inhalateur ; disponible auprès de fournisseurs comme par exemple GlaxoSmithKline sous le nom de Serevent®) soulage l'allodynie mécanique chez les souris neuropathiques (« Cuff ») après traitement chronique. Pour effectuer ce traitement, les animaux ont été placés de façon individuelle à l'intérieur de chambres d'inhalation (disponible auprès de fournisseurs comme par exemple GlaxoSmithKline, chambre d'inhalation Babyhaler® référence 7072184) dont les sorties d'air principales ont été obturées. Le flacon pressurisé de Servent® est connecté à la chambre et deux pressions permettent de délivrer 50 µg de salmétérol par séance d'administration (soit 100 µg par jour) à l'intérieur de la chambre d'inhalation lorsque le museau de la souris est proche de-l'inhalateur. L'animal est alors laissé 5 minutes dans la chambre avant d'être retiré et remis dans sa cage. Le traitement a débuté 2 semaines après la chirurgie de pose des manchons autour du nerf sciatique. Après 2 semaines de traitement avec deux pressions d'inhalateur par séance d'administration, le traitement a été passé pour neuf jours à quatre pressions d'inhalateur par séance d'administration. Le graphe de la figure 16 correspond aux résultats avant chirurgie, avant le début du traitement et au dernier jour de traitement. Les animaux contrôles pour le traitement (animaux non-traités) suivent la même procédure mais sans délivrance de salmétérol.

Ces données montrent pour la première fois qu'une administration de salmétérol par inhalation peut soulager la douleur neuropathique, notamment l'allodynie neuropathique.

### Exemple 14: THERAPIE : L'agoniste des béta2-adrénorécepteurs terbutaline soulage l'allodynie neuropathique.

Le graphe de la figure 17 montre qu'un traitement par l'agoniste béta2 adrénergique terbutaline (5 mg/kg, injection 2 fois par jour (matin et soir)) (terbutaline hémisulfate ; disponible auprès de fournisseurs comme par exemple Sigma-Aldrich numéro de catalogue T2528 ; dilué dans une solution saline à 0,9% de NaCl) supprime totalement l'allodynie mécanique après traitement chronique.

Les graphes de la figure 18 montrent qu'un traitement par l'agoniste béta2 adrénergique terbutaline (0,5 mg/kg, injection 2 fois par jour (matin et soir)) supprime totalement l'allodynie mécanique de la patte droite (patte avec chirurgie portant le « manchon » ou « cuff ») après traitement chronique sans affecter de façon aspécifique la réponse de la patte gauche non neuropathique (patte sans chirurgie).

Le graphe de la figure 19 montre qu'après 3 semaines de traitement par la terbutaline à la dose 0,5 mg/kg (injection 2 fois par jour (matin et soir)), si on co-administre la terbutaline et l'antagoniste des béta2-adrénorécepteurs ICI 118,551 (« ICI », 2 mg/kg, intrapéritonéal, tel que décrit dans l'exemple 5), cela fait réapparaître l'allodynie chez les animaux neurbpathiques (« Cuff ») en quelques jours. Le graphe montre le seuil-nociceptif des animaux après 3 semaines de traitement par la terbutaline et après 4 jours de co-traitement avec l'ICI 118,551. L'effet thérapeutique de la terbutaline passe donc bien par son action sur les béta2-adrénorécepteurs.

La sensibilité nociceptive des souris du groupe témoin (ou « sham ») n'est pas affectée par les injections d'ICI 118,551.

Les graphes de la figure 20 montrent que des traitements par l'agoniste béta2 adrénergique terbutaline aux doses 0,25 mg/kg ou 0,125 mg/kg (injection 2 fois par jour (matin et soir)) suppriment totalement l'allodynie mécanique de la patte droite (patte neuropathique) après traitement chronique.

Les graphes de la figure 21 récapitulent les données sur l'étude dose-réponse de l'action antiallodynique de la terbutaline. Ils présentent la variation de seuil nociceptif en % de la sensibilité en début d'expérience. Les données sont moyennées sur des mesures faites lors de la troisième semaine de traitement des animaux. La sensibilité mécanique des animaux traités par un placébo (solution saline, indiquée comme dose 0) reste stable lors de l'expérience. Les animaux neuropathiques (« cuff ») ont une allodynie mécanique de la patte droite qui porte le manchon. Le traitement chronique (3 semaines) des animaux neuropathiques par l'agoniste béta2 adrénergique terbutaline aux doses 0,125 mg/kg, 0,25 mg/kg, 0,5 mg/kg ou 5 mg/kg supprime l'allodynie de la patte droite neuropathique sans affecter la sensibilité mécanique de la patte gauche non-neuropathique. Ces mêmes traitements n'affectent pas les animaux témoins (« sham »). Pour comparatif, l'effet de l'antidépresseur nortriptyline (« Nor », 5 mg/kg, 2 fois par jour (matin et soir)) est également présenté. L'antidépresseur n'altère pas non plus la sensibilité d'animaux témoins. Le modèle et les tests utilisés sont ceux décrits dans l'exemple 1.

La sensibilité nociceptive des souris du groupe témoin (ou « sham ») n'est pas affectée par les injections de terbutaline.

Ces données montrent pour la première fois que l'administration de terbutaline peut soulager la douleur neuropathique, notamment l'allodynie neuropathique.

Le graphe de la figure 22 montre qu'un traitement par l'agoniste béta2 adrénergique terbutaline administré par application cutanée (0,1 mL de gel appliqué 2 fois par jour (matin et soir)) (2,5 mg/mL de terbutaline hémisulfate dans un gel contenant 5% d'hydroxyéthylcellulose dilué dans 5 % d'éthanol et 95 % eau. Hydroxyéthylcellulose disponible auprès de fournisseurs comme par exemple Fluka numéro de catalogue 54290) soulage l'allodynie mécanique chez les souris neuropathiques (« Cuff ») après traitement chronique. Le dos des animaux a été rasé pour que le pelage ne s'oppose pas à la pénétration du gel. Le gel est déposé sur le dos au niveau lombaire chez l'animal éveillé et le dépôt est massé doucement jusqu'à pénétration du produit. Les animaux contrôles pour le traitement suivent la même procédure, mais aucun agoniste béta2 adrénergique n'a été mis dans le gel. Le traitement par la terbutaline a débuté 8 jours après la chirurgie de pose des manchons autour du nerf sciatique, les autres paramètres expérimentaux sont semblables à ceux exposés dans l'exemple 1.

Ces données montrent pour la première fois qu'une administration de terbutaline par application cutanée peut soulager la douleur neuropathique, notamment l'allodynie neuropathique.

Le graphe de la figure 23 montre qu'un traitement par l'agoniste béta2 adrénergique terbutaline délivré par pose d'un implant sous-cutané (implant de tubulure silicone, disponible auprès de fournisseurs comme par exemple Sedat numéro de catalogue 602 235) contenant 10 mg de terbutaline hémisulfate soulage l'allodynie mécanique chez les souris neuropathiques (« Cuff »). L'implant permet de modéliser l'administration continue de modes d'administration comme par exemple les dispositifs transdermiques. Le tube de silicone de diamètre interne 1,4732 mm et de diamètre externe 1,9558 mm est coupé en tronçons de 1,5 cm de long. Une des extrémités est obturée avec de la colle silicone (Silastic ® Medical Adhesive Silicone Type A ; disponible auprès de fournisseurs comme par exemple Dow Corning numéro de catalogue 891). Après séchage, les tronçons sont remplis avec 10 mg de Terbutaline hémisulfate chacun, la poudre est tassée, le tronçon recoupé à environ 1 cm de longueur finale et est obturé avec la colle silicone. L'implant est implanté chez la souris sous anesthésie gazeuse à l'halothane. Sous anesthésie, la nuque et le haut du dos de l'animal sont rasés, une incision est faite dans le haut de la nuque et l'implant est glissé en sous-cutané en utilisant un guide canule. Les animaux contrôles pour le traitement subissent la même procédure avec la mise en place d'un implant vide. Les implants ont été mis en place 14 jours après la chirurgie de pose des manchons autour du nerf sciatique comme exposé dans l'exemple 1.

Ces données montrent pour la première fois qu'une administration continue de terbutaline peut soulager la douleur neuropathique, notamment l'allodynie neuropathique.

### Exemple 15 : Absence d'effet de l'injection aiguë (première injection) des agonistes des béta2-adrénorécepteurs fénotérol, salbutamol, salmétérol et terbutaline.

Le graphe de la figure 24 montre que la première injection des agonistes béta2 adrénergique fénotérol (0,7 mg/kg, intrapéritonéal), salbutamol (2 mg/kg, intrapéritonéal), salmétérol (1 mg/kg, intrapéritonéal), ou terbutaline (5 mg/kg, intrapéritonéal) tels que décrits dans les exemples précédants n'a pas d'effet sur l'allodynie mécanique des souris neuropathiques (« Cuff ») depuis 2 semaines.

Les souris du groupe témoin (ou « sham ») ne sont pas non plus affectées par la première administration de ces agonistes.

### Exemple 16 : THERAPIE : Récapitulatif, effet des agonistes béta-adrénergiques chez les animaux neuropathiques.

Le graphe de la figure 25 présente la variation de seuil nociceptif en % de la sensibilité en début d'expérience. Les données sont moyennées sur des mesures faites lors de la troisième semaine de traitement des animaux.

Les animaux neuropathiques (« Manchon » ou « cuff ») traités par un placébo (solution saline) maintiennent la forte baisse de leur seuil nociceptif, ce qui illustre la présence d'une allodynie (douleur).

Le traitement chronique (14-21 jours) des animaux neuropathiques par les agonistes béta-adrénergiques bambutérol, clenbutérol, fénotérol, formotérol (0,5 mg/kg), salbutamol, salmétérol, terbutaline (5 mg/kg), isoprénaline, ritodrine, métaprotérénol ou procatérol, tels que décrits dans les exemples précédants, soulagent tous cette allodynie.

Pour comparatif, l'effet de l'antidépresseur nortriptyline est également présenté. Les doses et modalités de traitement sont celles décrites ci-dessus dans les exemples 2, 7, 9 à 14 et 20. Le modèle et les tests utilisés sont ceux décrits dans l'exemple 1.

Les données présentées ici démontrent pour la première fois qu'un traitement chronique par des agonistes béta ou béta2 adrénergique administrés seuls permet de soulager l'allodynie neuropathique.

### Exemple 17 : Récapitulatif, effet des agonistes béta-adrénergiques chez des animaux témoins.

Le graphe de la figure 26 présente la variation de seuil nociceptif en % de la sensibilité en début d'expérience. Les données sont moyennées sur des mesures faites lors de la troisième semaine de traitement des animaux.

Les animaux témoins (« Manchon » ou « cuff ») sont traités par un placébo (solution saline). Leur sensibilité mécanique reste stable lors de l'expérience.

Le traitement chronique (14-21 jours) des animaux neuropathiques par les agonistes béta-adrénergiques bambutérol, clenbutérol, fénotérol, formotérol (0,5 mg/kg), salbutamol, salmétérol, terbutaline (5 mg/kg), isoprénaline, ritodrine, métaprotérénol ou procatérol, n'affectent pas les animaux témoins. Pour comparatif, l'effet de l'antidépresseur nortriptyline est également présenté. L'antidépresseur n'altère pas non plus la sensibilité d'animaux témoins. Les doses et modalités de traitement sont celles décrites ci-dessus dans les exemples 2, 7, 9 à 14 et 20. Le modèle et les tests utilisés sont ceux décrits dans l'exemple 1.

Ces données montrent qu'un traitement chronique par des agonistes béta ou béta2 adrénergique administrés seuls n'affecte pas la sensibilité d'animaux témoins.

### Exemple 18 : Effet d'un blocage des béta2-adrénorécepteurs sur l'action du bambutérol, du fénotérol, du formotérol et du salmétérol

Les graphes de la figure 27 montrent qu'après 3 semaines de traitement par les agonistes béta-adrénergiques bambutérol, fénotérol, formotérol ou salmétérol, si on co-administre ces agonistes et l'antagoniste des béta2-adrénorécepteurs ICI 118,551 (« ICI », 2 mg/kg, intrapéritonéal), cela fait réapparaître l'allodynie chez les souris neuropathiques (« Cuff ») en quelques jours. Les graphes montrent le seuil nociceptif des animaux après 3 semaines de traitement par les agonistes et après 3 à 6 jours de co-traitement avec l'ICI 118,551. Les doses et modalités de traitement sont celles décrites ci-dessus dans les exemples 5, 9 à 11 et 13. Le modèle et les tests utilisés sont ceux décrits dans l'exemple 1.

L'effet thérapeutique de ces agonistes passe donc bien par leur action sur les béta2-adrénorécepteurs.

La sensibilité nociceptive des souris du groupe témoin (ou « sham ») n'est pas affectée par les injections d'ICI 118,551.

### Exemple 19 : Effet d'un blocage des béta2-adrénorécepteurs sur l'action du salbutamol.

Les graphes de la figure 28 montrent qu'après 3 semaines de traitement par l'agoniste béta2-adrénergique salbutamol, si on co-administre cet agoniste et l'antagoniste des béta2-adrénorécepteurs ICI 118,551, cela fait réapparaître l'allodynie chez les souris neuropathiques (« Cuff ») en quelques jours. Le graphe de gauche montre le seuil nociceptif des animaux après 3 semaines de traitement par le salbutamol et après 3 jours de co-traitement avec l'ICI 118,551 co-injecté par voie intrapéritonéale (2 mg/kg). Le graphe de droite montre le seuil nociceptif des animaux après au moins 3 semaines de traitement par le salbutamol et après 2 injections de co-traitement avec l'ICI 118,551 administré par voie intrathécale avant chaque injection de salbutamol (2 mg/kg, intrapéritonéal). Les doses et modalités de traitement sont celles décrites ci-dessus dans les exemples 5 et 12 pour les injections intrapéritonéales et ci-dessous pour la description de la procédure d'injection intrathécale. Le modèle et les tests utilisés sont ceux décrits dans l'exemple 1. L'effet thérapeutique du salbutamol passe donc bien par son action sur les béta2-adrénorécepteurs.

Les résultats de l'injection intrathécale montrent que les béta2-adrénorécepteurs spinaux et/ou ceux des ganglions rachidiens sont importants pour l'effet thérapeutique du salbutamol.

La procédure d'injection intrathécale (i.t.) de l'antagoniste des béta2-adrénorécepteurs ICI 118,551 (3 µg dans 10 µL) est réalisée sous anesthésie gazeuse à l'halothane selon le protocole décrit par Inoue et coll. (Nature Medicine 2004, 10 :712-718 **(12)).** Ces injections i.t. sont réalisées juste avant l'injection intrapéritonéale de salbutamol (2 mg/kg, i.p.). Une aiguille d'injection de gauge 27 connectée à une seringue Hamilton de 50 µL est insérée entre les vertèbres L₅ et L₆ dans l'espace sous-arachnoidal. Le placement correct de l'aiguille est vérifié par la présence d'un mouvement réflexe de la queue de la souris. Les souris sont pré-testées avant cette procédure de co-traitement, puis co-traitées deux fois (un matin et le soir correspondant) avant d'être retestées le lendemain matin pour leur sensibilité nociceptive mécanique.

La sensibilité nociceptive des souris du groupe témoin (ou « sham ») n'est pas affectée par les injections d'ICI 118,551, que celles-ci soient intrapéritonéales ou intrathécales.

### Exemple 20 : Effet d'une stimulation des béta ou béta2-adrénorécepteurs : autres tests.

Dans les conditions décrites dans les exemples ci-dessus, on teste les molécules suivantes :
- le bitoltérol (par exemple entre 0,05 et 5 mg/kg, 2 fois par jour, intrapéritonéal) ;
- l'isoprénaline ou isoprotérénol (par exemple entre 0,05 et 5 mg/kg, 2 fois par jour, intrapéritonéal) ;
- le lévalbutérol (par exemple entre 0,05 et 5 mg/kg, 2 fois par jour, intrapéritonéal) ;
- le métaprotérénol (par exemple entre 0,05 et 5 mg/kg, 2 fois par jour, intrapéritonéal) ;
- le pirbutérol (par exemple entre 0,25 et 50 mg/kg, 2 fois par jour, intrapéritonéal) ;
- le procatérol (par exemple entre 0,05 et 5 mg/kg, 2 fois par jour, intrapéritonéal) ;
- le réprotérol (par exemple entre 0,05 et 5 mg/kg, 2 fois par jour, intrapéritonéal) ;
- la ritodrine (par exemple entre 0,25 et 50 mg/kg, 2 fois par jour, intrapéritonéal) ;
- le tulobutérol (par exemple entre 0,05 et 5 mg/kg, 2 fois par jour, intrapéritonéal).

Le graphe de la figure 29 montre qu'un traitement par l'agoniste béta adrénergique isoprénaline (0,5 mg/kg, injection 2 fois par jour (matin et soir)) (DL-isoprotérénol chloride ; disponible auprès de fournisseurs comme par exemple Sigma-Aldrich numéro de catalogue 15627 ; dilué dans une solution saline à 0,9% de NaCl) supprime totalement l'allodynie mécanique chez les souris neuropathiques (« Cuff ») après traitement chronique.

Ces données montrent pour la première fois que l'administration d'isoprénaline peut soulager la douleur neuropathique, notamment l'allodynie neuropathique

Le graphe de la figure 30 montrent qu'après 3 semaines de traitement par l'agoniste béta-adrénergique isoprénaline, si on co-administre l'antagoniste des béta2-adrénorécepteurs ICI 118,551 (« ICI », 2 mg/kg, intrapéritonéal), cela fait réapparaître l'allodynie chez les souris neuropathiques (« Cuff ») en quelques jours. Le graphe montre le seuil nociceptif des animaux après 3 semaines de traitement par l'isoprénaline et après 4 jours de co-traitement avec l'ICI 118,551. Le modèle et les tests utilisés sont ceux décrits dans l'exemple 1.

L'effet thérapeutique de cet agoniste passe donc bien par son action sur les béta2-adrénorécepteurs.

La sensibilité nociceptive des souris du groupe témoin (ou « sham ») n'est pas affectée par les injections d'ICI 118,551.

Le graphe de la figure 31 montre qu'un traitement par l'agoniste béta2 adrénergique ritodrine (10 mg/kg, injection 2 fois par jour (matin et soir)) (ritodrine hydrochloride ; disponible auprès de fournisseurs comme par exemple Sigma-Aldrich numéro de catalogue R0758 ; dilué dans une solution saline à 0,9% de NaCl contenant 0,3% d'antioxydant acide ascorbique) supprime totalement l'allodynie mécanique chez les souris neuropathiques (« Cuff ») après traitement chronique.

Ces données montrent pour la première fois que l'administration de ritodrine peut soulager la douleur neuropathique, notamment l'allodynie neuropathique.

Le graphe de la figure 32 montre qu'après 3 semaines de traitement par l'agoniste béta-adrénergique ritodrine, si on co-administre l'antagoniste des béta2-adrénorécepteurs ICI 118,551 (« ICI », 2 mg/kg, intrapéritonéal), cela fait réapparaître l'allodynie chez les souris neuropathiques (« Cuff ») en quelques jours. Le graphe montre le seuil nociceptif des animaux après 3 semaines de traitement par la ritodrine et après 4 jours de co-traitement avec l'ICI 118,551. Le modèle et les tests utilisés sont ceux décrits dans l'exemple 1.

L'effet thérapeutique de cet agoniste passe done bien par son action sur les béta2-adrénorécepteurs.

Le graphe de la figure 33 montre qu'un traitement par l'agoniste béta2 adrénergique métaprotérénol (1 mg/kg, injection 2 fois par jour (matin et soir)) (métaprotérénol hémisulfate ; disponible auprès de fournisseurs comme par exemple Sigma-Aldrich numéro de catalogue M2398 ; dilué dans une solution saline à 0,9% de NaCl) supprime totalement l'allodynie mécanique chez les souris neuropathiques (« Cuff ») après traitement chronique.

Ces données montrent pour la première fois que l'administration de métaprotérénol peut soulager la douleur neuropathique, notamment l'allodynie neuropathique.

Le graphe de la figure 34 montre qu'un traitement par l'agoniste béta2 adrénergique métaprotérénol administré par application cutanée (0,1 mL de gel appliqué 2 fois par jour (matin et soir)) (1 mg/mL de métaprotérénol hémisulfate dans un gel contenant 5% d'hydroxyéthylcellulose dilué dans 5 % d'éthanol et 95 % eau. Hydroxyéthylcellulose disponible auprès de fournisseurs comme par exemple Fluka numéro de catalogue 54290) soulage l'allodynie mécanique chez les souris neuropathiques (« Cuff ») après traitement chronique. Le dos des animaux a été rasé pour que le pelage ne s'oppose pas à la pénétration du gel. Le gel est déposé sur le dos au niveau lombaire chez l'animal éveillé et le dépôt est massé doucement jusqu'à pénétration du produit. Les animaux contrôles pour le traitement suivent la même procédure, mais aucun agoniste béta2 adrénergique n'a été mis dans le gel. Le traitement par le métaprotérénol a débuté 8 jours après la chirurgie de pose des manchons autour du nerf sciatique, les autres paramètres expérimentaux sont semblables à ceux exposés dans l'exemple 1.

Ces données montrent pour la première fois qu'une administration métaprotérénol par application cutanée peut soulager l'allodynie neuropathique.

Le graphe de la figure 35 montre qu'un traitement par l'agoniste béta2 adrénergique procatérol (0,8 mg/kg, injection 2 fois par jour (matin et soir)) (procatérol hydrochloride ; disponible auprès de fournisseurs comme par exemple Biotrend AG numéro de catalogue BN0432 ; dilué dans une solution saline à 0,9% de NaCl) supprime totalement l'allodynie mécanique chez les souris neuropathiques (« Cuff ») après traitement chronique.

Ces données montrent pour la première fois que l'administration de procatérol peut soulager la douleur neuropathique, notamment l'allodynie neuropathique.

### Liste des références

**(1)** Bouhassira D, Lantéri-Minet M, Attal N, Laurent B, Touboul C. Prevalence of chronic pain with neuropathic characteristics in the general population. Pain 2008, 136:380-387.
**(2)** Merksey H, Bogduk N, éditeurs. Classification of Chronic Pain. IASP Press, Seattle 1994.
**(3)** McQuay HJ, Tramèr M, Nye BA, Caroll D, Wiffen PJ, Moore RA. A systemic review of antidepressants in neuropathic pain. Pain 1996, 68:217-227.
**(4)** Hempenstall K, Nurmikko TJ, Johnson RW, A'Hern RP, Rice ASC. Analgesic therapy in postherpetic neuralgia: a quantitative systematic review. PLoS Medicine 2:e164.
**(5)** Gilron I, Watson CP, Cahill CM, Moulin DE. Neuropathic pain: a practical guide for the clinician. CMAJ 2006, 175:265-275.
**(6)** Attal N, Cruccu G, Haanpaa M, Hansson P, Jensen TS, Nurmikko T, Sampaio C, Wiffen P, EFNS Task Force. European Journal of Neurology 2006, 13:1153-1169.
**(7)** Moulin DE, Clark AJ, Gilron I, Ware MA, Watson CP, Sessle BJ, Coderre T, Morley-Foster PK, Stinson J, Boulanger A, Peng P, Finley GA, Taenzer P, Squire P, Dion D, Cholkan A, Gilani A, Gordon A, Henry J, Jovey R, Lynch M, Mailis-Gagnon A, Panju A, Rollman GB, Velly A, Canadian Pain Society. Pharmacological management of chronic neurotahic pain - consensus and guidelines from the Canadian pain Society. Pain Research Management 2007, 12:13-21.
**(8)** Dworkin RH, O'Connor AB, Backonja M, Farrar JT, Finerup NB, Jensen TS, Kalso EA, Loeser JD, Miaskowski C, Nurmikko TJ, Portenoy RK, Rice ASC, Stacey BR, Treede RD, Turk DC, Wallace MS. Pharmacological management of neuropathic pain: evidence-based recommendation. Pain 2007, 132:237-251.
**(9)** Crassous PA, Denis C, Paris H, Sénard JM. Interest of alpha2-adrenergic agonists and antagonists in clinical practice : background, facts and perspectives. Current Topics in Medicinal Chemistry 2007, 7:187-194.
**(10)** Benbouzid M, Gavériaux-Ruff C, Yalcin I, Waltisperger E, Tessier LH, Muller A, Kieffer BL, Freund-Mercier MJ, Barrot M. Delta-opioid receptors are critical for tricyclic antidepressant treatment of neuropathic allodynia. Biological Psychiatry 2008, 63:633-636.
**(11)** Benbouzid M, Pallage V, Rajalu M, Waltisperger E, Doridot S, Poisbeau P, Freund-Mercier MJ, Barrot M. Sciatic nerve cuffing in mice: a model of sustained neuropathic pain. European Journal of Pain 2008, 12:591-599.
**(12)** Inoue M, Rashid MH, Fujita R, Contos JJ, Chun J, Ueda H. Initiation of neuropathic pain requires lysophosphatidic acid receptor signalling. Nature Medicine 2004; 10:712-718.

## Revendications

1. Agoniste béta-2 adrénergique comme principe actif pour utilisation dans le traitement de l'allodynie neuropathique chronique, ledit agoniste béta-2 adrénergique étant administré de manière chronique.

2. Agoniste béta-2 adrénergique comme principe actif pour utilisation selon la revendication 1, ledit agoniste béta-2 adrénergique étant choisi dans le groupe comprenant bambutérol, bitolterol, clenbutérol, fénotérol, formotérol, isoprotérénol, levalbutérol, métaprotérénol, pirbutérol, procatérol, réprotérol, ritodrine, salbutamol, salmétérol, terbutaline, ou tulobutérol.

3. Agoniste béta-2 adrénergique comme principe actif pour utilisation selon la revendication 1 ou 2, ledit agoniste béta-2 adrénergique étant administré une, deux ou trois fois par jour.

4. Agoniste béta-2 adrénergique comme principe actif pour utilisation selon l'une quelconque des revendications 1 à 3, ledit agoniste béta-2 adrénergique étant administré à une dose comprise entre 0,01 et 20 mg/jour.

5. Agoniste béta-2 adrénergique comme principe actif pour utilisation selon l'une quelconque des revendications 1 à 3, ledit agoniste béta-2 adrénergique étant administré à une dose comprise entre 0,05 et 15 mg/jour.

6. Agoniste béta-2 adrénergique comme principe actif pour utilisation selon l'une quelconque des revendications 1 à 3, ledit agoniste béta-2 adrénergique étant administré à une dose comprise entre 0,05 et 10 mg/jour.

7. Agoniste béta-2 adrénergique comme principe actif pour utilisation selon l'une quelconque des revendications 1 à 3, ledit agoniste béta-2 adrénergique étant administré à une dose comprise entre 0,1 et 5 mg/jour.

8. Agoniste béta-2 adrénergique comme principe actif pour utilisation selon l'une quelconque des revendications 1 à 7, ledit agoniste béta-2 adrénergique étant à usage humain.

9. Agoniste béta-2 adrénergique comme principe actif pour utilisation selon l'une quelconque des revendications 1 à 7, ledit agoniste béta-2 adrénergique étant à usage vétérinaire.

10. Agoniste béta-2 adrénergique comme principe actif pour utilisation selon l'une quelconque des revendications 1 à 9, ledit agoniste béta-2 adrénergique étant administré sous une forme choisie dans le groupe comprenant une forme injectable, un sirop, une solution buvable, un comprimé, un comprimé sécable, un comprimé pelliculé, un comprimé pelliculé sécable, un comprimé gastrorésistant, un comprimé enrobé, un comprimé dispersable, un comprimé à croquer, une plaquette thermoformée, une gélule, un effervescent, un comprimé effervescent sécable, une poudre pour solution buvable, des granules pour suspension buvable, une suspension pour inhalation, une poudre pour inhalation, une solution pour inhalation par nébuliseur, une poudre pour inhalation en gélule, un suppositoire, un collyre, une crème, une pommade, un gel, un spray, un dispositif transdermique ou patch, une pastille, une solution pour perfusion intraveineuse, pour administration péridurale ou intra- ou péri-articulaire ou périphérique ou régionale ou paravertébrale ou intrathécale ou infiltration.

## Claims

1. Beta2-adrenergic agonist as an active principle for use in the treatment of chronic neuropathic allodynie, said beta2-adrenergic agonist being administered chronically.

2. Beta2-adrenergic agonist as an active principle for use according to claim 1, said beta2-adrenergic agonist being chosen from the group comprising bambuterol, bitolterol, clenbuterol, fenoterol, formoterol, isoproterenol, levalbuterol, metaproterenol, pirbuterol, procaterol, reproterol, ritodrin, salbutamol, salmeterol, terbutalin and tulobuterol.

3. Beta2-adrenergic agonist as an active principle for use according to claim 1 or 2, said beta2-adrenergic agonist being administered once, twice or three times a day.

4. Beta2-adrenergic agonist as an active principle for use according to any one of claims 1 to 3, said beta2-adrenergic agonist being administered at a dose of between 0.01 and 20 mg/day.

5. Beta2-adrenergic agonist as an active principle for use according to any one of claims 1 to 3, said beta2-adrenergic agonist being administered at a dose of between 0.05 and 15 mg/day.

6. Beta2-adrenergic agonist as an active principle for use according to any one of claims 1 to 3, said beta2-adrenergic agonist being administered at a dose of between 0.05 and 10 mg/day.

7. Beta2-adrenergic agonist as an active principle for use according to any one of claims 1 to 3, said beta2-adrenergic agonist being administered at a dose of between 0.1 and 5 mg/day.

8. Beta2-adrenergic agonist as an active principle for use according to any one of claims 1 to 7, said beta2-adrenergic agonist being for human use.

9. Beta2-adrenergic agonist as an active principle for use according to any one of claims 1 to 7, said beta2-adrenergic agonist being for veterinary use.

10. Beta2-adrenergic agonist as an active principle for use according to any one of claims 1 to 9, said beta2-adrenergic agonist being administered in a form chosen from the group comprising an injectable form, a syrup, an oral solution, a tablet, a breakable tablet, a film-coated tablet, a breakable film-coated tablet, a gastroresistant tablet, a coated tablet, a dispersible tablet, a chewable tablet, a thermoformed pack, a hard gelatin capsule, an effervescent product, a breakable effervescent tablet, a powder for oral solution, granules for oral suspension, a suspension for inhalation, a powder for inhalation, a solution for inhalation by nebulizer, a powder for inhalation in a hard gelatin capsule, a suppository, a collyrium, a cream, an ointment, a gel, a spray, a transdermal patch or patch, a pastil, a solution for intravenous perfusion, for peridural or intra- or peri-articular or peripheral or regional or paravertebral or intrathecal administration or infiltration.

## Patentansprüche

1. Beta 2-adrenergischer-Agonist als aktives Prinzip zur Verwendung in der Behandlung von chronischer neuropathischer Allodynie, wobei besagter Beta 2-adrenergischer-Agonist chronisch verabreicht wird.

2. Beta 2-adrenergischer-Agonist als aktives Prinzip zur Verwendung nach Anspruch 1, wobei besagter Beta 2-adrenergischer-Agonist innerhalb der Gruppe gewählt wird, zu der Bambuterol, Bitolterol, Clenbuterol, Fenoterol, Formoterol, Isoproterenol, Levalbuterol, Metaproterenol, Pirbuterol, Procaterol, Reproterol, Ritodrin, Salbutamol, Salmeterol, Terbutalin oder Tulobuterol gehören.

3. Beta 2-adrenergischer-Agonist als aktives Prinzip zur Verwendung nach Anspruch 1 oder 2, wobei besagter Beta 2-adrenergischer-Agonist ein- zwei- oder dreimal täglich verabreicht wird.

4. Beta 2-adrenergischer-Agonist als aktives Prinzip zur Verwendung nach einem beliebigen der Ansprüche 1 bis 3, wobei besagter Beta 2-adrenergischer-Agonist in einer Dosis von 0,01 bis 20 mg/Tag verabreicht wird.

5. Beta 2-adrenergischer-Agonist als aktives Prinzip zur Verwendung nach einem beliebigen der Ansprüche 1 bis 3, wobei besagter Beta 2-adrenergischer-Agonist in einer Dosis von 0,05 bis 15 mg/Tag verabreicht wird.

6. Beta 2-adrenergischer-Agonist als aktives Prinzip zur Verwendung nach einem beliebigen der Ansprüche 1 bis 3, wobei besagter Beta 2-adrenergischer-Agonist in einer Dosis von 0,05 bis 10 mg/Tag verabreicht wird.

7. Beta 2-adrenergischer-Agonist als aktives Prinzip zur Verwendung nach einem beliebigen der Ansprüche 1 bis 3, wobei besagter Beta 2-adrenergischer-Agonist in einer Dosis von 0,1 bis 5 mg/Tag verabreicht wird.

8. Beta 2-adrenergischer-Agonist als aktives Prinzip zur Verwendung nach einem beliebigen der Ansprüche 1 bis 7, wobei besagter Beta 2-adrenergischer-Agonist zur humanen Verwendung bestimmt ist.

9. Beta 2-adrenergischer-Agonist als aktives Prinzip zur Verwendung nach einem beliebigen der Ansprüche 1 bis 7, wobei besagter Beta 2-adrenergischer-Agonist zur veterinären verwendung bestimmt ist.

10. Beta 2-adrenergischer-Agonist als aktives Prinzip zur Verwendung nach einem beliebigen der Ansprüche 1 bis 9, wobei besagter Beta 2-adrenergischer-Agonist in einer Form verabreicht wird, die aus der Gruppe gewählt wird, zu der gehören: Injektionsform, ein Sirup, eine orale Suspension, eine Tablette, eine teilbare Tablette, eine filmbeschichtete Tablette, eine teilbare filmbeschichtete Tablette, eine magensaftresistente Tablette, eine beschichtete Tablette, eine dispergierbare Tablette, eine Kautablette, eine Blisterpackung, eine Kapsel, eine Brausetablette, eine teilbare Brausetablette, ein Pulver für eine orale Suspension, ein Granulat für eine orale Suspension, eine Suspension zur Inhalation, ein Pulver zur Inrialation, eine Lösung zur Inhalation durch einen Vernebler, ein Inhalationspulver in einer Kapsel, ein Zäpfchen, Augentropfen, eine Creme, eine Salbe, Gel, Spray, ein transdermales Pflaster oder Pflaster, eine Pastille, eine Lösung zur intravenösen Infusion, eine zur periduralen oder intra- oder periartikularen oder peripheren oder regionalen oder paravertebralen oder intrathekalen Verabreichung oder Infiltration.
